# EUROPÄISCHE PATENTSCHRIFT

(11) **EP 0 611 373 B1**
(45) Veröffentlichungstag und Bekanntmachung des Hinweises auf die Patenterteilung: **26.03.1997**
(21) Anmeldenummer: 92924466.3
(22) Anmeldetag: 25.05.1992
(51) Int. Cl.: C07H 21/00, C07H 19/10, C07H 19/20, A61K 31/715, A61K 31/70

(54) **OLIGO-2'-DESOXYNUKLEOTIDE UND IHRE VERWENDUNG ALS ARZNEIMITTEL MIT ANTIVIRALER WIRKSAMKEIT**
OLIGO-2'-DEOXYNUCLEOTIDES AND THEIR USE AS DRUGS WITH AN ANTIVIRAL ACTION
OLIGO-2'-DESOXYNUCLEOTIDES ET LEUR UTILISATION COMME MEDICAMENTS A ACTIVITE ANTIVIRALE

(30) Priorität: 25.05.1991 DE 4117186
(43) Veröffentlichungstag der Anmeldung: 24.08.1994
(73) Patentinhaber: BOEHRINGER MANNHEIM GMBH, 68298 Mannheim (DE)
(72) Erfinder: MÜHLEGGER, Klaus, D-8121 Polling (DE); VON DER ELTZ, Herbert, D-8120 Weilheim (DE); SEELA, Frank, D-4500 Osnabrück (DE); ROSEMEYER, Helmut, D-4500 Osnabrück (DE)
(74) Vertreter: Fouquet, Herbert, Dr.
(86) Internationale Anmeldenummer: EP9201172
(87) Internationale Veröffentlichungsnummer: WO9221692

(56) Entgegenhaltungen:
- Nucleosides and Nucleotides, Band 9, Nr. 4, 24. August 1990, (New York, US) N. I. Sokolova et al.: 'Dinucleoside phosphates containing arabinose or deoxyxylose. Hydrolysis by exonucleases and stacking properties', Seiten 515- 531
- Journal of Biochemistry, Band 98, 1985, (Tokoyo, JP) C. Nakayama et al.: 'Differential inhibitory effects of 5-substituted 1-B-D-xylofuranosyluracil 5'-
- Journal of the American Chemical Society, Band 105, 1983, (Gaston, PA, US) F. Hansske et al.: 'A deoxygenative (1,2)-hydride shift rearrangement converting cyclic cis-diol monotosylates to inverted secondaryalcohols', Seite n 6736- 6737, (in der Anmeldung erwähnt)
- Helvetica Chimica Acta, Band 74, Nr. 4, 1991, (Basel, CH) H. Rosemeyer et al.: '72. 1-(2'-deoxy-B-D-xylofuranosyl) thymine building blocks for solid-phase synthesis and properties of oligo(2'-deoxyxylonucleotides)', Seiten 748-760
- Helvetica Chimica Acta, Band 74, Nr. 8, 1991, (Basel, CH) H. Rosemeyer et al.: '192. 9-(2'-deoxy-B-D-xylofuranosyl)adenine building blocks for solid-phase synthesis and properties of oligo(2'-deoxyxylonucleotides)', Seiten 2054-2067

## Beschreibung

Oligonukleotide, deren Sequenz komplementär zur RNA oder DNA einer viralen Sequenz oder zu einem Onkogen sind, sind für die Therapie von Virusinfektionen von potentiellem Interesse, da sie die Expression viraler Gene inhibieren können. Das zugrundliegende, als Antisense-Prinzip bezeichnete, Verfahren ist beispielsweise in Zamecnik, P.C. und Stephenson, M.L. (1978) Proc. Natl. Acad. Sci. USA 75, 280 beschrieben.

Es hat sich jedoch gezeigt, daß beim Einschleusen von solchen Antisense-Oligonukleotiden in Zellen diese Oligonukleotide bevorzugt durch Spaltung von Phosphodiester-Brücken durch zelleigene Enzyme rasch abgebaut und damit unwirksam werden.

Es ist daher nicht an Versuchen gefehlt, gegen enzymatischen Abbau resistente Antisense-Oligonukleotide zu synthetisieren (Uhlmann, E. und Peyman, A. (1990) in "Antisense Oligonucleotides: A new therap. Principle", Chem. Rev. 90, 543 - 584). Solche Modifikationen wurden bisher in erster Linie an den Internukleotid-Brücken, d. h. am Phosphoratom vorgenommen. So sind z. B. Oligonukleosid-phosphothioate und -phosphodithioate, sowie nichtionische Oligonukleosid-methylphosphonate, -methylphosphothioate, -alkylphosphotriester und -alkylphosphoramidate beschrieben, die gegen enzymatischen Abbau resistent sind. Ein Nachteil dieser Verbindungen liegt z. B. in ihrer Chiralität bzgl. des Phosphoratoms. Dies bedeutet, daß jeweils zwei Diastereoisomerenpaare existieren. Diese Uneinheitlichkeit begrenzt jedoch ihre pharmakologische Wirksamkeit bzw. erfordert eine aufwendige Trennung der Isomeren vor dem Einsatz als Therapeutika.

Eine weitere bekannte Verbindungsklasse, die zur Antisense-Therapie vorgeschlagen wurde, sind Oligonukleotide, welche intercalierende oder reaktive Liganden enthalten. So wurden beispielsweise Acridin-modifizierte Oligomere oder quervernetzbare (Psoralen-, Azidoproflavin-substituierte) Oligonukleotide beschrieben (Hélène, C. and Thuong, N. T. in "Antisense RNA and DNA", Curr. Commun. Mol. Biol.; Cold Spring Harbor Laboratory, Cold Spring Harbor NY, 1987). Die Herstellung solcher Oligonukleotide ist jedoch sehr aufwendig.

Weiter ist bekannt, als Antisense-Oligonukleotide solche Oligodesoxynukleotide zu verwenden, die einen konfigurativ veränderten glykonischen Teil besitzen (alpha-DNA). Eine derartige alpha-DNA, welche ausschließlich aus alpha-D-konfigurierten Purin- und Pyrimidin-2'-desoxynukleosiden besteht, wird von zelleigenen Enzymen nicht oder nur sehr langsam abgebaut und wäre deshalb für die Antisense-Therapie geeignet. Ein Nachteil dieser Verbindungen ist jedoch die sehr aufwendige und umständliche Synthese. (Cohen, J.S. in Topics in Molecular and Structural Biology, "Oligonucleotides: Antisense Inhibitors of Gene Expression", MacMillan Press, Ltd. 1989). Ferner sind Dinukleotid-monophosphate bekannt, die bedingt durch einen threo-konfigurierten Baustein Nukleaseresistenz aufweisen (Sokolova et al.; Nucleosides and Nucleotides 9/4 (1990), 515-531). Entsprechende Oligonukleotide sind jedoch weder offenbart, noch wären diese ohne weiteres herstellbar. Zudem sind die Dinukleotide nicht zur Hybridisierung und Ausbildung von Duplexen geeignet.

Aufgabe der vorliegenden Erfindung war es deshalb, Oligonukleotide zur Verfügung zu stellen, die in eukaryontischen Zellen nicht enzymatisch abgebaut werden, einfach herstellbar und als antivirale Arzneimittel nach dem Antisense-Prinzip geeignet sind.

Gegenstand der Erfindung sind deshalb Oligodesoxyribonukleotide, in denen jeweils am 5'- und 3'-Ende mindestens zwei 2'-Desoxy-β-D-erythro-pentofuranosyl-Gruppen durch 2'-Desoxy-β-D-threo-pentofuranosyl-Gruppen ersetzt sind, und die aus 6 bis 100 Nukleotidbausteinen bestehen.

Ein weiterer Gegenstand der Erfindung sind Oligodesoxyribonukleotide, in denen mindestens 20 % der 2'-Desoxy-β-D-erythro-pentofuranosyl-Gruppen in aufeinanderfolgenden Nukleotidbausteinen durch 2'-Desoxy-β-D-threo-pentofuranosylGruppen ersetzt sind und die aus 6 - 100 Nukleotidbausteinen bestehen.

Überraschenderweise sind derartige Oligonukleotide (im weiteren auch als Oligonukleotide bezeichnet) resistent oder weitgehend resistent gegen den nukleolytischen Abbau durch zelluläre Enzyme, wie beispielsweise Phosphodiesterasen, Exo- und Endonukleasen. Sie bilden außerdem mit natürlichen 2'-Desoxyribonukleotiden stabile doppelsträngige Hybridstrukturen unter den natürlichen Bedingungen in eukaryontischen Zellen aus, obwohl sie vermutlich, zumindest teilweise, linkshelicale DNA-Strukturen besitzen, wie ihre CD-Spektren belegen. Die erfindungsgemäßen Oligonukleotide sind geeignet, die Expression von viralen Genen und Onkogenen in eukaryontischen Zellen zu inhibieren und sind deshalb als Antisense-Oligonukleotide therapeutisch einsetzbar.

Vorzugsweise sind 30 %, besonders bevorzugt alle, der 2'-Desoxy-β-D-erythro-pentofuranosyl-Gruppen durch 2'-Desoxy-β-D-threo-pentofuranosyl-Gruppen ersetzt.

Wenn in einem Oligonukleotid alle 2'-Desoxy-β-D-erythropentofuranosylgruppen durch 2'-Desoxy-β-D-threo-pentofuranosylgruppen ersetzt sind, wird dieses Oligonukleotid zweckmäßig als Oligodesoxyxylonukleotid bezeichnet.

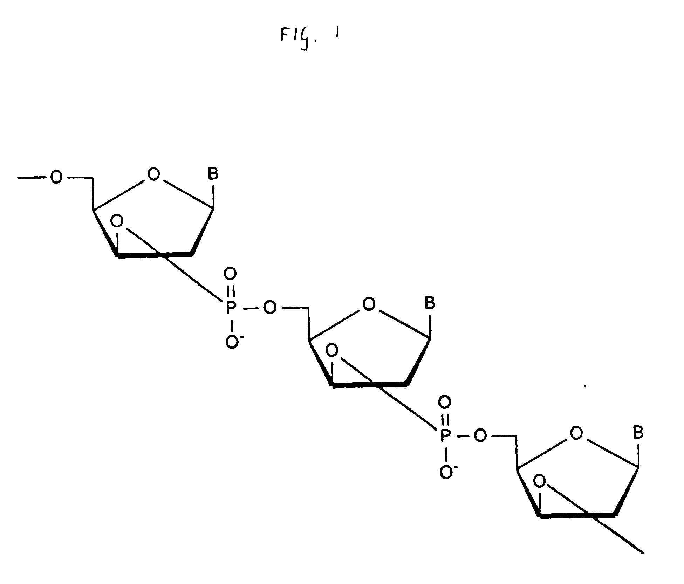

Der Aufbau eines erfindungsgemäßen Oligonukleotids ist in Fig. 1 schematisch (Ausschnitt aus einem Oligodesoxyxylonukleotid) gezeigt.

Im weiteren werden die Nukleotide, die 2'-Desoxy-β-D-erythropentofuranosyl-Gruppen enthalten, als 2'-Desoxyribonukleotide und die Nukleotide, die 2'-Desoxy-β-D-threo-pentafuranosyl-Gruppen enthalten, als 2'-Desoxyxylonukleotide bzw.-Bausteine bezeichnet.

Außerdem werden 2'-Desoxyribonukleotid-Bausteine als dB (z.B. dA, dT, dC, dG) und 2'-Desoxyxylonukleotid-Bausteine als dxB (z.B. dxA,dxT,dxC,dxG) bezeichnet.

Es ist bevorzugt, daß die dxBs und dBs in den erfindungsgemäßen Oligonukleotiden aufeinanderfolgend in Blöcken vorliegen.

Bevorzugte erfindungsgemäße Oligonukleotide sind demnach:
d {xBxB(B)ₙxBxB}
d {(B)ₘ(xB)ₙ(B)ₒ}
d {(xB)ₘ(B)ₙ(xB)ₒ}
wobei n, m und o mindestens 4 ist, mit der Maßgabe, daß die Gesamtlänge des erfindungsgemäßen Oligonukleotides 100 Nukleotidbausteine nicht überschreitet.

In einer ebenfalls bevorzugten Ausführungsform können einer oder mehrere Nukleotidbausteine dB an spezifischen Positionen des Oligonukleotids (z.B. Erkennungssequenzen von Endonukleasen) durch dxB ersetzt sein um die Spaltung durch Endonukleasen zu verhindern.

Als Basen sind alle natürlichen oder modifizierten Nukleobasen geeignet. Besonders bevorzugte modifizierte Basen sind 5-Methylcytosin oder Desazapurine, wie 1-Desazaadenin, 3-Desazaadenin, 7-Desazaadenin, 1-Desazaguanin, 3-Desazaguanin, 7-Desazaguanin, 1-Desazahypoxanthin, 3-Desazahypoxanthin, 7-Desazahypoxanthin und solche Basen, die am C-5 der Pyrimidine, am C-7 im Falle von 7-Desazapurinen oder am C-8 der Purine substituiert sind.

Zusätzlich können die erfindungsgemäßen Oligonukleotide Modifikationen an den Internukleotidbrücken enthalten, wobei die Modifikationen bevorzugt an allen Internukleotidbrücken des erfindungsgemäßen Oligonukleotids vorliegen. Bevorzugt hierbei sind Phosphothioate, Methylphosphonate und Phosphoamidate.

Am 3'- und/oder 5'-Ende können die erfindungsgemäßen Oligonukleotide alle dem Fachmann bekannten geeigneten Endgruppen enthalten. Bevorzugt ist für das 3'-Ende und für das 5'-Ende Wasserstoff, Mono-, Di- oder Triphosphat, eine Reportergruppe oder eine Intercalatorgruppe . Auch die anderen Nukleotidbausteine können durch Reportergruppen oder Intercalatorgruppen modifiziert sein.

Unter einer Reportergruppe im Sinne der Erfindung ist ein Hapten wie z.B. Biotin oder Digoxigenin oder ein Fluoreszenzfarbstoffrest zu verstehen. Geeignete Intercalatorgruppen sind bei Hélène, C., loc. cit. beschrieben und sind vorzugsweise Phenanthrolin, Acridin, Actinomycin bzw. dessen Chromophor oder Schwermetall-Komplexbildner wie EDTA. Ebenso vorteilhaft sind solche Gruppen, die zur Vernetzung von Nukleinsäuren führen, wie z.B. Psoralen.

Erfindungsgemäße Oligonukleotide bestehen vorzugsweise aus 15 bis 30 Nukleotidbausteinen. Die Basenfolge in den erfindungsgemäßen Oligonukleotiden richtet sich nach der Sequenz des Virus oder Onkogens gegen die das Oligonukleotid gerichtet sein soll. So sind beispielsweise zur Therapie von HIV I-Infektionen erfindungsgemäß Oligonukleotide besonders geeignet, bei denen alle Basen A oder T darstellen und alle 2'-Desoxy-β-D-erythro-pentofuranosyl-Gruppen durch 2'-Desoxy-β-D-threo-pentofuranosyl-Gruppen ersetzt sind, da die virale Sequenz von HIV I mehrere Cluster mit Poly A- bzw. Poly T-Sequenzen enthält. Weitere besonders bevorzugte Oligonukleotide sind solche, die zu bestimmten, für die Replikation viraler Gene wichtigen Genombereichen komplementär sind. Dies sind z. B. für das HIV-Genom der regulatorische Bereich des rev Gens (Matsukura, M. et al. (1989) Proc. Natl. Acad. Sci. USA, 86, 4244)

Die Herstellung der erfindungsgemäßen Oligonukleotide erfolgt in an sich bekannter Weise, beispielsweise nach der Phosphattriester-, Phosphittriester- oder H-Phosphonat-Methode in homogener Phase oder an einem Träger. Bevorzugt werden die beiden letzteren Verfahren eingesetzt, wobei die Synthese üblicherweise mit automatisch arbeitenden Synthesegeräten erfolgt.

Ein weiterer Gegenstand der Erfindung ist deshalb ein Verfahren zur Herstellung von Oligodesoxyribonukleotiden, in denen jeweils am 5'- und 3'-Ende mindestens zwei 2'-Desoxy-β-D-erythro-pentofuranosyl-Gruppen durch 2'-Desoxy-β-D-threo-pentofuranosylgruppen ersetzt sind, und die aus 6 bis 100 Nukleotidbausteinen bestehen, nach dem Verfahren der Oligonukleotidsynthese, wobei ein Startnukleosid an einen festen Träger gebunden wird und anschließend durch schrittweise Kupplung mit entsprechend aktivierten monomeren Nukleotidbausteinen das gewünschte Oligonukleotid aufgebaut wird, gegebenenfalls während und nach der Synthese dreiwertiger Phosphor zu fünfwertigem Phosphor oxidiert wird, das Oligonukleotid vom Träger mit einer ersten Base, heterocyklische Schutzgruppen mit einer zweiten Base, die 5'-Schutzgruppe mit einer Säure abgespalten und das Oligonukleotid ggf. aufgereinigt wird.

Ein weiterer Gegenstand der Erfindung ist ein Verfahren zur Herstellung von Oligodesoxyribonukleotiden, in denen mindestens 20 % der 2'-Desoxy-β-D-erythro-pentofuranosyl-Gruppen durch 2'-Desoxy-β-D-threo-pentofuranosyl-Gruppen in aufeinanderfolgenden Nukleotidbausteinen ersetzt sind, und die aus 6 bis 100 Nukleotidbausteinen bestehen, nach dem Verfahren der Oligonukleotidsynthese, wobei ein Startnukleosid an einen festen Träger gebunden wird und anschließend durch schrittweise Kupplung mit entsprechend aktivierten monomeren Nukleotidbausteinen das gewünschte Oligonukleotid aufgebaut wird, gegebenenfalls während oder nach der Synthese dreiwertiger Phosphor zu fünfwertigem Phosphor oxidiert wird, das Oligonukleotid vom Träger mit einer Base, heterocyklische Schutzgruppen mit einer zweiten Base, die 5'-Schutzgruppe mit einer Säure abgespalten und das Oligonukleotid ggf. aufgereinigt wird.

Vorzugsweise werden Oligonukleotide hergestellt, die aus 15 bis 30 Nukleotidbausteinen bestehen.

Die Oxidation des dreiwertigen Phosphors zu fünfwertigen Phospor erfolgt bei Verwendung der Phosphoramidite als Nukleotidbausteine zweckmäßig nach jeder Kupplung eines Nukleotidbausteins, bei Verwendung der Phosphonate als Nukleotidbausteine zweckmäßig nach Aufbau des gesamten Oligonukleotids. Vorzugsweise wird zur Oxidation Jod (z.B. Jod/H₂O/Lutidin) oder, für die Herstellung der 2'Desoxyxylonukleosid-3'-phosphothioate und -3'-phosphodithioate, Schwefelungsreagenzien (z.B. Schwefel in Pyridin/ Schwefelkohlenstoff) verwendet.

Zur alkalischen Spaltung wird vorzugsweise Ammoniak bei 60°C, zur Entfernung der 5'-Schutzgruppe vorzugsweise 80 %ige wäßrige Essigsäure oder Tetrabutylammoniumfluorid, wenn eine Silyl-Schutzgruppe verwendet wird, bei Raumtemperatur verwendet.

Nach Basen- und Säurespaltung wird zweckmäßigerweise neutralisiert und aufgereinigt. Zur Aufreinigung wird vorzugsweise eine reverse phase HPLC oder Anionentauscher-HPLC verwendet, wobei anschließend entsalzt wird.

Die Durchführung derartiger Oligonukleotidsynthesen sind dem Fachmann allgemein bekannt und beispielsweise in Gait, M.J. in "Oligonucleotide synthesis, a practical approach", IRL Press, LTD. 1984, Narang, S.A., "Synthesis and application of DNA and RNA", Academic Press 1987, beschrieben.

Das Trägermaterial besteht aus dem Fachmann bekannten anorganischen (Controlled Pore Glas, Fractosil®) oder organischpolymerem Material (z. B. Polystyrol).

Zur Herstellung der erfindungsgemäßen Oligonukleotide werden vorzugsweise an das Trägermaterial über ein Kopplungsreagenz (vgl. z.B. Gait, M.J. loc.cit.) ein monomeres 2'-Desoxyxylonukleosid oder 2'-Desoxyribonukleosid gekoppelt, welches als Startnukleosid für die Oligonukleotidsynthese dient.

Ein weiteres Verfahren zur Herstellung der erfindungsgemäßen Oligonukleotide ist die Phosphattriestermethode (vgl. Gait, M.J. loc.cit.).

Ein weiterer Gegenstand der Erfindung sind basen- und zuckergeschützte 2'-Desoxyxylonukleosid-3'-phosphoamidite, -3'-H-phosphonate und -P-methyl-phosphoamidite. Diese Verbindungen sind als Nukleotidbausteine zur Herstellung der erfindungsgemäßen Oligonukleotide geeignet.

Besonders bevorzugt enthalten die erfindungsgemäßen Nukleotidbausteine die Basen Adenin, Guanin, Cytosin, Thymidin, Uracil, 5-Methyl-cytidin oder Desazapurine, wie z. B. 1-Desazaadenin, 3-Desazaadenin, 7-Desazaadenin, 1-Desazaguanin, 3-Desazaguanin und 7-Desazaguanin. Weitere bevorzugte Basen sind solche, die am C-5 der Pyrimidine, am C-7 der 7-Desazapurine oder am C-8 der Purine substituiert sind.

Die erfindungsgemäßen Nukleotidbausteine enthalten vorzugsweise Schutzgruppen an den heterocyclischen Basen, 5'-OH- oder 3'-OH-Schutzgruppen.

Als Schutzgruppen an den heterocyclischen Basen werden vorzugsweise Aminoschutzgruppen verwendet wie z.B. die Benzoyl-, Formamidin-, Isobutyryl- oder die Phenoxyacetyl-Gruppe.

Als 5'-OH-Schutzgruppe des Zuckerteils werden vorzugsweise eine Triphenylmethyl-, Monomethoxytrityl-, Dimethoxytrityl, t-Butyl-dimethylsilyl-, t-Butyldiphenylsilyl-, t-Butylmethoxyphenylsilyl- oder Pixylgruppe verwendet.

Als Phosphoramidite sind 3'-O-(2-Cyanoethyl)-N, N-Diisopropyl-aminophosphane und die 3'-O-Methyl-N, N-diisopropylamino-phosphane bevorzugt. Die H-Phosphonate werden vorzugsweise als Salze eingesetzt.

In einer besonders bevorzugten Ausführungsform sind die erfindungsgemäßen Nukleotidbausteine ³²P oder ³⁵S-markiert.

Die erfindungsgemäßen kupplungsfähigen Nukleotidbausteine werden aus den entsprechenden 2'-Desoxyxylonukleosiden (Herstellung beispielsweise analog Fox und Miller J. Org. Chem. 28 (1963) 936, Hansske und Robins J. Am. Chem.Soc. 105 (1983) 6736-6737 oder F. Seela und H.P. Muth, Helvetica Chimica Acta 74 (1991) 1081 - 1090) hergestellt.

Die Herstellung der monomeren Nukleotidbausteine erfolgt nach den dem Fachmann geläufigen Methoden, beispielsweise wie sie in Gait, M.J. loc.cit beschrieben sind.
Die erfindungsgemäßen Oligonukleotide können durch Ligation mit weiteren erfindungsgemäßen oder bekannten Oligonukleotiden am 3'- und/oder 5'-Ende verlängert werden. Derartige Ligationsreaktionen mittels DNA- oder RNA-Ligase sind dem Fachmann geläufig und z. B. in Maniatis et al., Molecular Cloning, A Laboratory Manual, Cold Spring Harbor Laboratory, New York (1982), beschrieben.

Weiterhin können auch die erfindungsgemäßen Oligonukleotide enzymatisch, unter Verwendung einer DNA- und/oder RNA-Polymerase, hergestellt werden. Hierbei werden Nukleotide der allgemeinen Formel 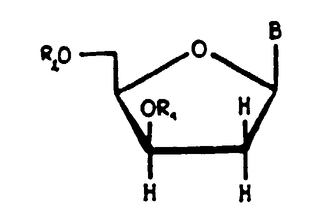 wobei B A, T, C, G oder eine Base aus der Gruppe 1-Desazaadenin, 3-Desazaadenin, 7-Desazaadenin, 1-Desazaguanin, 3-Desazaguanin, 7-Desazaguanin, 1-Desazahypoxanthin, 3-Desazahypoxanthin, 7-Desazahypoxanthin, an C-7 substituierte 7-Desazapurine, an C-8 substituierte Purine, C-5 substituierte Pyrimidine enthält, R₁ ein Wasserstoffatom oder eine Reportergruppe oder eine Intercalatorgruppe und R₂ ein Mono-, Di oder Triphosphat darstellt.

Ein weiterer Gegenstand der Erfindung sind daher Nukleotide der allgemeinen Formel 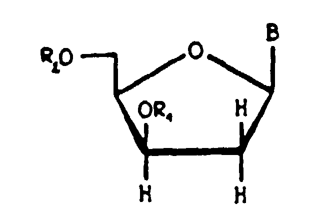 wobei B A, T, C, G oder eine Base aus der Gruppe 1-Desazaadenin, 3-Desazaadenin, 7-Desazaadenin, 1-Desazaguanin, 3-Desazaguanin, 7-Desazaguanin, 1-Desazahypoxanthin, 3-Desazahypoxanthin, 7-Desazahypoxanthin, an C-7 substituierte 7-Desazapurine, an C-8 substituierte Purine, C-5 substituierte Pyrimidine enthält, R₁ ein Wasserstoffatom oder eine Reportergruppe oder eine Intercalatorgruppe und R₂ ein Mono-, Di oder Triphosphat darstellt.

Ein weiterer Gegenstand der Erfindung ist die Verwendung von Oligodesoxyribonukleotiden, in denen jeweils am 5'- und 3'-Ende mindestens zwei 2'-Desoxy-β-D-erythro-pentafuranosyl-Gruppen durch 2'-Desoxy-β-D-threo-pentafuranosyl-Gruppen ersetzt sind, und aus 6 bis 100 Nukleotidbausteinen bestehen, zur Herstellung eines Arzneimittels mit antiviraler Wirksamkeit.

Ein weiterer Gegenstand der Erfindung sind Oligodesoxyribonukleotide, in denen mindestens 20 % der 2'-Desoxy-β-D-erythro-pentofuranosyl-Gruppen in aufeinanderfolgenden Nukleotidbausteinen durch 2'-Desoxy-β-D-threo-pentofuranosylGruppen ersetzt sind und die aus 6 - 100 Nukleotidbausteinen bestehen, zur Herstellung eines Arzneimittels mit antiviraler Wirksamkeit.

Die erfindungsgemäßen Oligonukleotide und ihre Salze können als Medikamente verwendet werden, z.B. in Form von pharmazeutischen Präparaten, die man oral, z.B. in Form von Tabletten, Dragees, Hart- oder Weichgelatinekapseln, Lösungen, Emulsionen oder Suspensionen verabreichen kann. Sie können auch rektal z.B. in Form von Suppositorien oder partenteral z.B. in Form von Injektionslösungen verabreicht werden. Für die Herstellung von pharmazeutischen Präparaten können diese Verbindungen in therapeutisch inerten organischen und anorganischen Trägern verarbeitet werden. Beispiele von solchen Trägern für Tabletten, Dragees und Hartgelatineklapseln sind Laktose, Maisstärke oder Derivate davon, Talg, Stearinsäure oder Salze davon. Geeignete Träger für die Herstellung von Lösungen sind Wasser, Polyole, Saccharose, Invertzucker und Glukose. Geeignete Träger für Injektionslösungen sind Wasser, Alkohole, Polyole, Glycerol und pflanzliche Öle. Geeignete Träger für Suppositorien sind pflanzliche und gehärtete Öle, Wachse, Fette und halbflüssige Polyole.

Die pharmazeutischen Präparate können auch Konservierungsmittel, Lösungsmittel, Stabilisierungsmittel, Netzmittel, Emmulgiermittel, Süßstoffe, Farbstoffe, Geschmacksmittel, Salze zur Veränderung des osmotischen Drucks, Puffer, Überzugsmittel oder Antioxydantien, sowie ggf. andere therapeutische Wirkstoffe enthalten.

Die folgenden Beispiele und Abbildungen erläutern die Erfindung weiter:
- Fig. 1: zeigt einen Ausschnitt aus einem Oligodesoxyxylonukleotid.
- Fig. 2: zeigt die Hydrolyse des Oligonukleotids d(GTAGAAxTxTCTAC) durch Kälbermilz-Phosphodiesterase (H = Hypochromizität des erhaltenen Endprodukts, die eingesetzte Figur zeigt das Elutionsprofil der HPLC des Endprodukts, a = (Et₃NH)OAc pH7/Acetonitril (95:5), b = 0 - 20 % Acetonitril in Laufmittel a.
- Fig. 3: zeigt die Schmelzkurve des d(A₁₂)/d (xT₁₂) Hybrids (b) im Vergleich zum Hybrid d (A₁₂)/d(T₁₂) (a) und den einzelsträngigen Oligonukleotiden d(A₁₂) (c) und d(T₁₂) (d).
- Fig. 4: zeigt das CD-Spektrum von d (T₁₂) (a), T (b), xT (c) und d (xT₁₂) (d)
- Fig. 5: zeigt das CD-Spektrum der Hybride d(A₁₂) / d(T₁₂) (a) und d(A₁₂) / d(xT₁₂) (c) sowie des einzelsträngigen Oligonukleotids d(A₁₂) (b)

### Beispiel 1

### 1-(2'-Desoxy-β-D-threo-pentofuranosyl)thymin

Die Synthese erfolgte nach Fox und Miller [J. Org. Chem. (1963), 28, 936].

Das Produkt (gelöst in (D₆)DMSO) wird durch folgende Signale im ¹H-NMR-Spektrum charakterisiert (Angaben von δ in ppm): 11.25 (s, br. NH); 7.80 (s, H-C(6)); 6.06 (dd, J(H-C(1'), H_{β}-C(2') = 2.7 Hz, J(H-C(1'), H_{α}-C(2') = 5.5 Hz, H-C(1')); 5.25 (3'-OH); 4.69 (5'-OH); 4.23 (m, H-C(3')); 3.76 (m, H-C(4')); 3.70 (m, H₂-C(5')); ∼ 2.5 (H_{α} -C(2')); 1.84 (d, J(H_{β}-C(2'),H_{α}-C(2') = - 16.0 Hz, H_{β}-C(2')); 1.66 (s, CH₃).

### Beispiel 2

### 1-[2'-Desoxy-5'-(4,4-dimethoxytriphenylmethyl)-β-D-threopentofuranosyl]-thymin

500 mg 1-(2'-Desoxy-β-D-threo-pentofuranosyl)thymin (2,06 mmol) wurden durch mehrfache Co-Evaporation mit wasserfreiem Pyridin (je 5 ml) getrocknet. Der ölige Rückstand wurde in 15 ml wasserfreiem Pyridin gelöst und nacheinander mit 1,0 g 4,4'-Dimethoxytriphenylmethylchlorid (3 mmol) 0,5 ml Diisopropyl-ethylamin (3 mmol) versetzt. Man rührt 3 h bei 40°C unter Stickstoff, gießt danach die Lösung in 50 ml 5%-iges wässriges NaHCO₃, und extrahiert 2x mit je 100 ml Dichlormethan. Die vereinigten organischen Phasen werden über Natriumsulfat getrocknet und das Lösungsmittel abgedampft. Nach mehrfachem Co-Evaporieren mit Toluol wird der Rückstand an Kieselgel 60H mittels Flash-Chromatographie auf gereinigt (Säule: 6x 15 cm, CH₂Cl₂-MeOH, 98:2). Die Fraktionen, welche das Hauptprodukt enthalten, werden eingeengt bis zum farblosen, schaumigen Rückstand (780 mg, 69% d.Th.).

DC (Kieselgel, CH₂Cl₂-MeOH, 95:5) R_{f} 0,6.

Das Produkt (gelöst in (D₆)DM_{SO}) wird durch folgende Signale im ¹H-NMR-Spektrum charakterisiert (Angaben von δ in ppm): 11.30 (s, NH); 7.62 (s, H-C(6)); 7.45-6.86 (m, 13H, aromat. H); 6.12 (dd, J = 6.2, 2.7 Hz, H-C(1')); 5.22 (d, J = 3.4 Hz, 3'-OH); 4.20 (m,H-C(3')); 4.10 (m, H-C(4')); 3.73 (s, 6H, 2x OCH₃); 3.40 und 3.19 (2m, H₂-C(5')); ∼ 2.51 (m, H_{α} -C(2')); 1.87 (d, J = -15.4 Hz, H_{β}-2'); 1.66 (s, CH₃).

Elementaranalyse für C₃₁H₃₂N₂O₇ (544,6): berechnet C 68,37, H 5,92, N 5,14; gefunden C 68,44, H 6,00, N 5,15.

### Beispiel 3

### 1-[(2'-Desoxy-β-D-threo-pentofuranosyl-5'-O-(4,4'-dimethoxytriphenylmethyl)]-thymin-(3'-H-phosphonat), Trietylamoniumsalz

Zu einer Lösung von 0,4 ml Phosphoroxytrichlorid (4,6 mmol) und 5,1 ml N-methylmorpholin (46 mmol) in 36 ml Dichlormethan wurden 1,06 g 1,2,4-Triazol (15,3 mmol) gegeben. Nach 30 minütigem Rühren wird die Lösung auf 0°C gekühlt und dazu eine Lösung von 500 mg 1-[2'-Desoxy-5'-(4,4'-dimethoxytriphenylmethyl)-β-D-threo-pentofuranosyl]-thymin in 12 ml Dichlormethan langsam zugefügt. Nach weiterem Rühren von 10 Minuten bei Raumtemperatur wird die Reaktionsmischung in 50 ml 1 M wässriges Triethylammoniumbicarbonatlösung, pH 8,0, eingegossen, durchgeschüttelt und die Phasen abgetrennt. Die wässrige Phase wird mit 30 ml CH₂Cl₂ extrahiert und die vereinigten organischen Extrakte über Na₂SO₄ getrocknet und zu einem farblosen Schaum eingedampft. Flash-Chromatographie an Kieselgel 60H (Säule: 6x 15 cm, erst mit CH₂Cl₂-Et₃N, 92:8, dann mit CH₂Cl₂-MeOH-Et₃N, 88:10:2) lieferte eine Hauptfraktion, die vereinigt und eingedampft wurde. Der Rückstand wurde in 15 ml CH₂Cl₂ gelöst und 2x mit 1 M wässriger Triethylammoniumbicarbonatlösung, pH 8,0, extrahiert, die organischen Phasen über Natriumsulfat getrocknet und eingedampft. Man erhält 480 mg (74% d.Th.) des gewünschten Produkts in Form eines farblosen Schaums.

DC (Kieselgel, CH₂Cl₂-MeOH-Et₃N, 88:10:2): R_{f} 0,3.

Das Produkt (gelöst in (D₆)DMSO) wird durch folgende Signale im ¹H-NMR-Spektrum charakterisiert (Angaben von δ in ppm): 11.30 (s, NH); 7.66 (s, H-C(6)); 7.63-6.86 (m, 13H, aromat. H), 6.13 (dd, J = 6.8, 2.8 Hz, H-C(1')); 5.76 und 5.28 (d, J = 119 Hz, PH); 4.62 (m, H-C(3')); 4.12 (m, H-C(4')); 3.73 (s, 6H, 2x OCH₃); 3.34 und 3.16 (m, H₂-C(5')); 2.73 (q, CH₃CH₂NH); ∼ 2.5 (m, H_{α}-C(2')); 2.14 (d, J = -15.2 Hz, H_{β}-C(2')); 1.66 (s, CH₃); 1.03 (t, CH₃CH₂NH).

³¹P-NMR ((D₆)DMSO): 0.88 ppm (¹J(P-H) = 587 Hz; ³J(P-H-4') = 8.8 Hz).

Elementaranalyse für C₃₇H₄₈N₃O₉P (709,8): berechnet C 62,61, H 6,82, N 5,92; gefunden C 62,81, H 7,00, N 5,99.

### Beispiel 4

### 1-[2'-Desoxy-5'-O-(4,4'-dimethoxytriphenylmethyl)-β-D-threopentofuranosyl]-thymin-3'-[(2-cyanoethyl)-N,N-diisopropyl phosphoramidit]

Zu einer Lösung von 100 mg 1-[2'-Desoxy-5'-(4,4'-dimethoxytriphenylmethyl)-β-d-threo-pentofuranosyl]-thymin (0,18 mmol) und 0,1 ml N-ethyldiisopropylamin (0,57 mmol) in trockenem Tetrahydrofuran werden 45 µl Chlor-β-cyanoethoxy-(N,N-diisopropylamino)phosphan (0,2 mmol) während 2 Minuten unter Stick- stoff bei Raumtemperatur zugetropft. Es wird 30 Minuten gerührt und danach die Reaktion durch Zufügen von 4 ml 5%-igem wässrigen NaHCO₃ gestoppt. Die Reaktionsmischung wird 2x mit je 5 ml CH₂Cl₂ extrahiert. Die vereinigten organischen Phasen werden über Natriumsulfat getrocknet und eingedampft. Flash-Chromatographie (Kieselgel 60H, Säule: 3x 6 cm, Ethylacetat-CH₂Cl₂-Et₃N, 45:45:10) liefert 2 teilweise überlappende Hauptfraktionen der Diastereomeren (95 mg, 72% d.Th.).

DC (Kieselgel, CH₂Cl₂-EtOAc-Et₃N, 45:45:10) R_{f} 0,7 und 0,6.

³¹P-NMR (CDCl₃): 148,5 ppm (in DC höher laufende Verbindung); 151,8 ppm (in DC niedriger laufende Verbindung).

### Beispiel 5

### 9-(2'-Desoxy-β-D-xylofuranosyl)adenin (2'-Desoxyxyloadenosin)

Die Verbindung wurde gemäß der Vorschrift von Hansske und Robins [J. Am. Chem. Soc. (1983), 105, 6736-6737] dargestellt.

Das Produkt (gelöst in (D₆)DMSO) wird durch folgende Signale im ¹H-NMR-Spektrum charakterisiert (Angaben von δ in ppm): 8.35 (s, H-C(8)); 8.15 (H-C(2)); 7.34 (s, br., NH₂); 6.25 (dd, J(H-C(1'),H_{α}-C(2')) = 2.2 Hz; J(H-C(1'),H_{β}-C(2')) = 8.7 Hz; H-C(1')); 5.97 (s, br. 3'-OH); 4,69 (s, br., 5'-OH); 4.33 (m, H-C(3')); 3.89 (m, H-C(4')); 3.67 (m, H₂-C(5')); 2.78 (m, H_{α}-C(2')); 2.25 (dd, J(H_{α}-C(2'),H_{β}-C(2')) = -14.5 Hz; H_{β}-C(2')).

### Beispiel 5a

### 2'-Desoxy-xylo-inosin

2'-Desoxy-xylo-adenosin (78 mg, 0,31 mmol) wird in 3 ml Wasser suspendiert und mit Adenosin Desaminase (Kalbsmilz, 50 µg) versetzt. Man rührt 2 h bei Raumtemperatur und dampft zur Trockne ein; Ausbeute 75 mg (97 %) farblose Kristalle vom Schmp. 208-210°C.

Das Produkt (gelöst in (D₆)DMSO) wird durch folgende Signale im ¹H-NMR-Spektrum charakterisiert (Angaben von δ in ppm): 12.4 (br, NH); 8.31 (s, H-C(8); 8.07 (s, H-C(2); 6.24 (dd, J = 8.8, 1.6 Hz, H-C(1'); 5.45 (d, J = 4.0 Hz, 3'-OH); 4.70 (m, 5'-OH); 4.36 (m, J = 3.5 Hz, H-C(3'); 3.92 (m, H-C(4'); 3.71 und 3.60 (2 m, H₂-C(5'); 2.76 (m, H_{α}-C(2'); 2.25 (d, J = -15 Hz, H_{β}-C(2').

¹³C-NMR ((D₆)DMSO): 156.7 (C-6); 147.8 (C-4); 145.9 (C-2); 139.1 (C-8); 124.0 (C-5); 85.6 (C-4'); 82.6 (C-1'); 69.1 (C-3'); 59.9 (C-5'); 41.1 (C-2').

Das UV-Spektrum (in MeOH) zeigt ein Absorptionsmaximum bei 249 nm (εₘₐₓ = 10.500).

| Elementaranalyse: | | | |
|---|---|---|---|
| berechnet: | C 47.62; | H 4.80; | N 22.21 |
| gefunden : | C 47.76; | H 4.98; | N 22.15. |

### Beispiel 6

### 9-[2'-Desoxy-5'-O-(4,4'-dimethoxytriphenylmethyl)-β-D-xylopentofuranosyl]-6 -[[(dimethylamino)methyliden]amino]adenin

Eine Lösung von 2'-Desoxy-xyloadenosin (502 mg, 2 mmol) in Dimethylformamid (10 ml) wurde 18 h mit Dimethylformamiddiethylacetal (1.7 ml, 10 mmol) bei Raumtemperatur gerührt und anschließend eingedampft [DC (Kieselgel, CH₂Cl₂-Aceton-Et₃N (20 : 10 : 1)) R_{f} 0.1]. Der ölige Rückstand (0.8 g) wurde ohne weitere Reinigung in Pyridin (50 ml) gelöst und auf die Hälfte des Volumens eingeengt. Nach Zugabe von 4,4'-Dimethoxytriphenylmethylchlorid (760 mg, 2.24 mmol) und Ethyldiisopropylamin (0.37 ml, 2.1 mmol) wurde 3.5 h bei Raumtemperatur gerührt. Nach Abdampfen des Pyridins und Nachdampfen mit Toluol (2x 30 ml) wurde der Rückstand in CH₂Cl₂-Aceton-Et₃N (20:10:1, v/v/v, 5 ml) gelöst und an Kieselgel 60 (6x 10 cm, 0.5 bar; Laufmittel: CH₂Cl₂-Aceton-Et₃N, 20:10:1) chromatographiert. Ausbeute: 520 mg (43%) farbloser Schaum.

DC (Kieselgel, CH₂Cl₂-Aceton, Et₃N, 20:10:1) R_{f} 0.48.

Das Produkt (gelöst in (D₆)DMSO) wird durch folgende Signale im ¹H-NMR-Spektrum charakterisiert (Angaben von δ in ppm): 8.93 (s, =CH-); 8.43 (s, H-C(8)); 8.34 (s, H-C(2)); 7.17-7.40 (m, DMT); 6.75-6.84 (m, DMT); 6.41 (m, H-C(1')); 5.75 (d, J = 5.0 Hz, 3'-OH); 4.33 (m, H-C(3')); 4.20 (m, H-C(4')); 3.7 (m, 2 CH₃ und H₂-C(5)); 3.20 und 3.13 (2 s, 2 OCH₃); 2.78 (m, H_{α} -C(2')); 2.3 (d, H_{β}-C(2')).

| Elementaranalyse: | | | |
|---|---|---|---|
| berechnet: | C 67.09; | H 5.96; | N 13,81 |
| gefunden: | C 66.89; | H 6.03; | N 13,64 |

### Beispiel 6a

### 9-(2'-Desoxy-β-D-threo-pentofuranosyl)N⁶-benzoyladenin

2'-Desoxy-xyloadenosin (100 mg, 0.40 mmol) wird in trockenem Pyridin (10 ml) suspendiert und auf ein Drittel seines Volumens eingedampft. Man fügt Trimethylchlorsilan (0.25 ml, 2 mmol) hinzu und läßt 15 Minuten bei Raumtemperatur rühren. Anschließend wird Benzoylchlorid (0.23 ml, 2 mmol) hinzugefügt und weitere 2 Stunden bei Raumtemperatur gerührt. Nach Kühlen auf 0°C und Zugabe von 0.5 ml H₂O wird nach weiteren 5 Minuten 25 %iges wäßriges Ammoniak hinzugefügt und weitere 30 Minuten bei Raumtemperatur gerührt. Nach Abdampfen des Pyridins wird der Rückstand in Wasser gelöst und mit Ethylacetat (10 ml) extrahiert. Die organische Phase wird eingedampft und mit Pyridin (3 ml) und 25 %igem wäßrigem Ammoniak (1 ml) versetzt. Nach einer Stunde wird eingedampft und an Kieselgel 60 (70 ml) mit dem Laufmittel Ethylacetat/Aceton/Ethanol/H₂O (18:3:2:2) chromatographiert, wobei ein Hauptprodukt eluiert wird. Die Ausbeute beträgt 25 mg (60 %), der Schmelzpunkt beträgt 175 - 177°C.

DC (Kieselgel, Ethylacetat/Aceton/Ethanol/H₂O, 18:3:2:2): R_{f} 0.4.

Das Produkt (gelöst in (D₆)DMSO) wird durch folgende Signale im ¹H-NMR-Spektrum charakterisiert (Angaben von δ in ppm): 11.2(br, NH); 8.77 (s. H-C(8); 8.70 (s, H-C(2); 8.05 und 7.60 (2 m, Benzoyl-H); 6.47 (d, H-C(1'); 5.55 (d, 3'-OH); 4.73 (t, 5'-OH); 4.42 (m, H-C(3'); 4.00 (m, H-C(4'); 3,74 (m, H₂-C(5'); 2.83 (m, H_{α} -C(2'); 2.38 (m, H_{β}-C(2').

| | | | | |
|---|---|---|---|---|
| Elementaranalyse: | berechnet: | C 57.46; | H 4.82; | N 19.71; |
| | gefunden: | C 57.36; | H 4.95; | N 19.64. |

### Beispiel 6b

### 9-[2'-Desoxy-5'-O-(4,4'-dimethoxytriphenyl)-β-D-threopentofuranosyl]-N⁶-benzoyl-adenin

N^{Bz}dxA (280 mg, 0.79 mmol) wird durch Abdampfen mit Pyridin (30 ml) getrocknet und der Rückstand in Pyridin (15 ml) gelöst. Man fügt 4,4'-Dimethoxytriphenylmethylchlorid (315 mg, 0.93 mmol) und Ethyldiisopropylamin (0.14 ml, 0.85 mmol) hinzu und rührt 3 Stunden bei Raumtemperatur. Anschließend wird eingedampft und mehrmals mit Toluol (50 ml) nachgedampft. Der Rückstand wird an Kieselgel 60 (60 ml) mit CH₂Cl₂/Aceton (12:5) eluiert, wobei eine Hauptzone erhalten wird, aus der nach Abdampfen des Lösungsmittels 428 mg (82 %) der Titelverbindung als farbloser Schaum erhalten werden.

DC (CH₂Cl₂/Aceton, 12:5): R_{f} 0.47.

Das Produkt (gelöst in (D₆)DMSO) wird durch folgende Signale im ¹H- NMR-Spektrum charakterisiert (Angaben von δ in ppm): 11.2 (br. NH); 8.77 (s, H-C(2); 8.07 - 6.77 (m, Benzoyl- und DMT-H); 6.53 (d, H-C(1'); 5.51 (d, 3'-OH); 4.37 (m, H-C(3'); 4.28 (m, H-C(4'); 3.72 (m, 2 OCH und H₂-C(5'); 2.79 (m, H_{α}-C(2'); um 2.5 (m, H_{β}-C(2').

| | | | | |
|---|---|---|---|---|
| Elementaranalyse: | berechnet: | C 69.39; | H 5.36; | N 10.65 |
| | gefunden: | C 69.48; | H 5.84; | N 10.51 |

### Beispiel 7

### 9-[2'-Desoxy-5'-O-(4,4'-dimethoxytriphenylmethyl)-β-D-xylopentofuranosyl]-6-[[(dimethylamino)methyliden] amino]adenin-3'(H-phosphonat) Triethylammoniumsalz

Zu einer Lösung von PCl₃ (360 µl, 4.1 mmol) und N-Methylmorpholin (4.5 ml, 41 mmol) in CH₂Cl₂ (35 ml) wird 1,2,4-Triazol (0.94 g, 13.6 mmol) hinzugefügt und die Reaktionsmischung 30 min bei Raumtemperatur gerührt. Nach Abkühlen auf 0°C wird eine Lösung des geschützten Nucleosids aus Beispiel 6 (480 mg, 0.79 mmol) in CH₂Cl₂ (10 ml) tropfenweise zugefügt und die Lösung 10 min bei Raumtemperatur gerührt. Anschließend wird das Reaktionsgemisch in Triethylammoniumbicarbonatpuffer (1 M, pH 8, 50 ml) gegossen, die Phasen getrennt und die wässrige Phase 2x mit CH₂Cl₂ (30 ml) extrahiert. Die vereinigten organischen Phasen werden über Na₂SO₄ getrocknet und eingedampft. Der Rückstand wird an Kieselgel 60 (6x 10 cm, 0.5 bar, Laufmittel: 600 ml CH₂Cl₂-Et₃N, 92:8; CH₂Cl₂-MeOH-Et₃N, 88:10:2) chromatographiert. Nach Eindampfen der Hauptzone wird der Rückstand in CH₂Cl₂ (15 ml) gelöst und 2x mit Et₃NH⁺HCO₃⁻ (1 M, pH 8, 20 ml) extrahiert. Die organische Phase wird über Na₂SO₄ getrocknet und eingedampft; Ausbeute 390 mg (64%) farbloser Schaum.

DC (Kieselgel, CH₂Cl₂-MeOH-Et₃N, 88:10:2): R_{f} 0.65.

Das Produkt (gelöst in (D₆)DM_{SO}) wird durch folgende Signale im ¹H-NMR-Spektrum charakterisiert (Angaben von δ in ppm): 10.5 (s, br, NH); 8.95 (s, =CH-); 8.45 (s, H-C(8)); 8.41 (s, H-C(2)); 7.39-6.75 (m, DMT); 6.79 und 5.29 (P-H); 6.45 (m, H-C(1')); 4.79 (H-C(3')); 4.28 (m, H-C(4')); 3.71 (m, 2 CH₃ und H₂-C(5)); 3.20 und 3.13 (2 s, 2 OCH₃); 2.96 (m, CH₂); 2.5 (m, H₂-C(2')); 1.10 (t, CH₃).

| Elementaranalyse | | | |
|---|---|---|---|
| berechnet: | C 62.08; | H 6.77; | N 12,67 |
| gefunden: | C 62.12; | H 6.90; | N 12.46 |

### Beispiel 7a

### 9-[2'-Desoxy-5'-O-(4,4'-demethoxytriphenylmethyl)-β-D-threopentofuranosyl]-N⁶-benzoyladenin-3'-(H-phosphonat) Triethylammoniumsalz

Zu einer Lösung von PCl₃ (136 µl, 1.56 mmol) und N-Methylmorpholin (1.72 ml, 15.6 mmol) in CH₂Cl₂ (13 ml) wird 1,2,4-Triazol (359 mg, 5.2 mmol) hinzugefügt. Die Lösung wird 30 Minuten bei Raumtemperatur gerührt, anschließend auf 0°C gekühlt und ds vollständig geschützte Nukleosid (DMT-N^{Bz}dxA, 200 mg, 0.3 mmol, gelöst in CH₂Cl₂ (8 ml)) hinzugefügt. Nach 30 minütigem Rühren bei Raumtemperatur gießt man die Lösung in 1 mol/l TBK-Puffer (pH 8, 20 ml) und trennt die organische Phase ab. Nach nochmaliger Extraktion der wäßrigen Phase mit Dichlormethan (30 ml) werden die vereinigten organischen Phasen über Na₂SO₄ getrocknet und eingedampft. Der Rückstand wird an Kieselgel 60 (50 ml) chromatographiert. Mit CH₂Cl₂- Triethylamin (92:8) werden 31 mg (16 %) des Ausgangsmaterials eluiert; das gewünschte H-Phosphonat wird CH₂Cl₂-Methanol-Triethylamin (88:5:2) eluiert. Nach Abziehen des Lösungsmittels wird der Rückstand in CH₂Cl₂ (30 ml) gelöst und mit 1 mol/l TBK-Puffer (pH 8, 30 ml) extrahiert. Nach Trocknen der organischen Phase über Na₂SO₄ wird eingedampft. Es werden 158 mg (64 %) farblosser Schaum erhalten.

DC (Kieselgel, CH₂Cl₂-MeOH-Et₃N; 88:5:2): R_{f} = 0.3.

³¹P-NMR ((D₆)DMSO): 1.37 ppm (¹J(P-H) = 594 Hz; ³J(P-H-3') = 8.7 Hz).

| Elementaranalyse: | | | |
|---|---|---|---|
| berechnet: | C 64.22; | H 6.25; | N 10.21 |
| gefunden: | C 64.47; | H 6.48; | N 10.51 |

### Beispiel 8

### 9-[2'-Desoxy-5'-O-(4,4'-dimethoxytriphenylmethyl)-β-D-xylopentofuranosyl]-6-[[(dimethylamino)methyliden] amino]adenin-3'-[2-(cyanoethyl)-N,N-diisopropylphosphoramidit]

Zu einer Lösung des geschützten Nucleosids aus Beispiel 6 (122 mg, 0.2 mmol) in trockenem THF (1.5 ml) wird Ethyldiisopropylamin (110 µl, 0.63 mmol) hinzugefügt. Anschließend wird unter N₂ Chlor-β-cyanoethyloxy-(N,N-diisopropylamino)phosphan (50 µl, 0.22 mmol) innerhalb von 2 min zugetropft. Nachdem das Reaktionsgemisch 30 min bei Raumtemperatur gerührt wurde, fügt man wässriges NaHCO₃ (5%, 4 ml) hinzu und extrahiert 2x mit CH₂Cl₂ (5 ml). Die vereinigten organischen Phasen werden über Na₂SO₄ getrocknet, eingedampft und der Rückstand an Kieselgel 60H (3x 6 cm, 0.5 bar, Laufmittel: CH₂Cl₂-EtOAc-Et₃N, 45:45:10) chromatographiert; Ausbeute: 156 mg (96%) farbloses Öl.

DC (Kieselgel, CH₂Cl₂-EtOAc-Et₃N, 45:45:10) R_{f} 0.50 und 0.53 (2 Diastereoisomere).

³¹P-NMR ((D₆)DMSO): 151.4, 146.7 ppm.

### Beispiel 9

### 1-[2'-Desoxy-5'-O-(4,4'-dimethoxytriphenylmethyl)-3'-O-succinyl-β-D-threo-pentofuranosyl]-thymin

Zu einer Lösung von 250 mg des Nucleosides aus Beispiel 2 (0,46 mmol) in 10 ml trockenem Pyridin werden 70 mg 4-Dimethylaminopyridin (0,54 mmol) und 230 mg Bernsteinsäureanhydrid (2,3 mmol) gegeben und die Mischung 70 h bei 40°C gerührt. Danach fügt man 3 ml Wasser zu dem Reaktionsgemisch und dampft bis zum trockenen Rückstand ein. Pyridinspuren werden durch Co-Evaporation mit Toluol entfernt. Das zurückbleibende Öl wird in CH₂Cl₂ gelöst und nacheinander mit 10%-iger wässriger Zitronensäure und Wasser ausgeschüttelt. Die organische Phase wird über Natriumsulfat getrocknet und eingedampft. Man nimmt den Rückstand in 2 ml eines Gemisches aus CH₂Cl₂ und Pyridin (95:5) auf und gießt die Lösung langsam unter Rühren in 50 ml eines Gemisches aus n-Pentan/Ether (1:1). Der ausgefallene Niederschlag wird abfiltriert und mittels Flash-Chromatographie an Kieselgel 60 (Säule: 10x 6 cm, Acetonitril-Wasser (9:1)) gereinigt. Man erhält als Hauptfraktion die gewünschte Verbindung (180 mg, 61% d.Th.) in Form eines farblosen Pulvers.

DC (Kieselgel, Acetonitril-Wasser, 9:1) R_{f} 0,8.

Das Produkt wird durch folgende Signale im ¹³C-NMR (Lösung in ((D₆)DMSO) charakterisiert (Angaben in ppm): 173.6, 171.2 (2 C=O); 163.8 (C-6); 158.3 (DMT); 150.5 (C-2); 144.7 (DMT); 135.5 (C-4); 135.4-126.9 (10 Signale, DMT); 113.3 (quart.-C, DMT); 109.0 (C-5); 85.8 (DMT); 83.6 (C-4'); 80.6 (C-1'); 72.3 (C-3'); 61.2 (C-5'); 55.1 (OCH₃, DMT); 40.6 (C-2'); 29.1, 29.0 (2 CH₂); 12.3 (CH₃-Thymin).

### Beispiel 10

### Fractosil-gebundenes 1-[2'-Desoxy-5'-O-(4,4'-dimethoxytriphenylmethyl)-3'-O-succinyl-β-D-threo pentofuranosyl]-thymin

Zu einer Lösung von 100 mg Nucleosid aus Beispiel 9 (0,16 mmol) in 1 ml eines Gemisches aus Dioxan-5% Pyridin werden 40 mg 4-Nitrophenol (0,29 mmol) und 60 mg N,N-Dicyclohexylcarbodiimid (0,3 mmol) unter Rühren bei Raumtemperatur gegeben. Nach 2 Stunden wird der abgeschiedene Dicyclohexylharnstoff durch Filtration entfernt und zum Filtrat 200 mg Fractosil® 200 (Merck, 450 µÄq. NH₂/g) und 1 ml Dimethylformamid zugefügt. Nach Zugabe von 0,2 ml Triethylamin wird die Suspension 4 Stunden bei Raumtemperatur geschüttelt. Dann werden 60 µl Essigsäureanhydrid zugegeben und das Schütteln weitere 30 min. fortgesetzt. Danach wird das Silicagel-gebundene Nukleosid abfiltriert, mit Dimethylformamid, Ethanol und Ether gewaschen und im Vakuum getrocknet.
Die Menge an Silicagel-gebundenem Nucleosid wurde wie folgt bestimmt: 5 mg des Fractosil-Trägers wurden mit 10 ml 0,1 M 4-Toluolsulfonsäure in Acetonitril behandelt. Der Überstand wurde bei 498 nm spektrophotometrisch ausgemessen, wobei 50,4 µmol Nucleosid pro g Fractosil gefunden wurden (ε_{DMT} = 70.000).

### Beispiel 10a

### Fractosil-gebundenes 9-[2'-Desoxy-5'-O-(4,4'-dimethoxytriphenylmethyl)-β-D-xylopentofuranosyl]-6 -[[(dimethylamino)methyliden]amino]adenin

Zu einer Lösung des vollständig geschützten Nukleosids aus Beispiel 6 (250 mg, 0.41 mmol) in Pyridin (10 ml) werden N,N-Dimethylaminopyridin (60 mg, 0.49 mmol) und Bernsteinsäureanhydrid (200 mg, 2 mmol) hinzugefügt und die Lösung 72 Stunden bei 40°C gerührt. Nach Zugabe von Wasser (3 ml) wird zur Trockne eingedampft und der Rückstand mit Toluol (50 ml) nachgedampft. Man löst in CH₂Cl₂ und extrahiert mit 10 %iger wäßriger Zitronensäurelösung (30 ml) und Wasser (30 ml). Die organische Phase wird über Na₂SO₄ getrocknet und eingedampft. Es werden 278 mg (96 %) farbloses Material erhalten, das ohne weitere Aufreinigung umgesetzt wird. Das Succinat (142 mg, 0.20 mmol) wird in einer 5 %igen Lösung von Pyridin in Dioxan (1.25 ml) gelöst und mit p-Nitrophenol (50 mg, 0.36 mmol) und Dicyclohexylcarbodiimid (80 mg, 0.40 mmol) bei Raumtemperatur versetzt. Man läßt 3 Stunden bei dieser Temperatur rühren und filtriert anschließend ausgefallenen Dicyclohexylharnstoff ab. Anschließend werden Dimethylformamid (1.25 ml) und Fractosil 200 (450 µeq NH₂/g) hinzugefügt. Nach Zugabe von Triethylamin (250 µl) wird die Suspension 4 Stunden bei Raumtemperatur geschüttelt und anschließend Essigsäureanhydrid (75 µl) hinzugefügt. Das Schütteln wird 30 Minuten fortgesetzt, das Silicagel-gebundene Nukleosid abfiltriert, mit Dimethylformamid, Ethanol und Ether gewaschen und im Vakuum getrocknet. Zur Bestimmung des Silikagel-gebundenen Nukleosids wurde wie unter Beispiel 10 beschrieben verfahren; Konzentration des Silikagel-gebundenen Nukleosids 27 µmol/g Fractosil.

### Beispiel 11

### Festphasensynthese des Oligonukleotides d(xT₁₂)

Die Synthese des Oligomeren geschah im 1 µMol-Maßstab mit dem 3'-Hydrogenphosphonat aus Beispiel 3. Die Synthese folgte dem Standardprotokoll des DNA-Synthesizers für die 3'-H-phosphonat-Methode (Applied Biosystems Users Manual des DNA-Synthesizers 380 B).
Die 4,4'-Dimethoxytrityl-Schutzgruppe des Oligomeren wurde durch Behandlung mit 80%-iger Essigsäure während 5 Minuten bei Raumtemperatur entfernt. Die Reinigung geschah durch HPLC an einer RP-18-Säule und einem Gradienten-System aus 0,1 M Et₃NHOHAc pH 7/Acetonitril (95:5) [A] und Acetonitril [B]. Das reine Oligomere wurde auf einer 4x 25 mm HPLC-Kartusche (RP-18-Silicagel) entsalzt und anschließend lyophilisiert.

### Beispiel 11 a

### Festphasensynthese des Oligonucleotides d(GTAGxAxAxCTAC)

Die Synthese des Oligonukleotides erfolgte im 1 µM-Maßstab mit den Nucleosid-3'-O-(2-Cyanoethyl)-N,N-diisopropylaminophosphoamiditen der Basen A, G, C und T, sowie der entsprechenden Phosphoamidite der 3'-Xylo-nucleoside xT und xA aus den Beispielen 4 und 8.Die Synthese folgte dem Standardprotokoll des DNA-Synthesizers für die Phosphoamidit-Methode (Applied Biosystems Users Manual des DNA-Synthesizers 380 B). Die Abspaltung der NH₂-Schutzgruppen erfolgte mit 25%iger, wässrigerAmmoniaklösung bei 60°C während 48 Stunden. Die Entfernung der 5'-O-Dimethoxytrityl-Schutzgruppen, die Reinigung über HPLC, sowie die Entsalzung erfolgte wie unter Beispiel 11 beschrieben.

### Beispiel 12

### Festphasensynthese weiterer Oligonukleotide

Die Festphasensynthese der Oligomeren d(xTCG xTCG CxTG xTCxT CCG CxTxT CxTxT CCxT GCC xA) und d(GxTxAGAATTCxTxAC) geschah in analoger Weise wie in Beispiel 11 angegeben.

### Beispiel 12 a

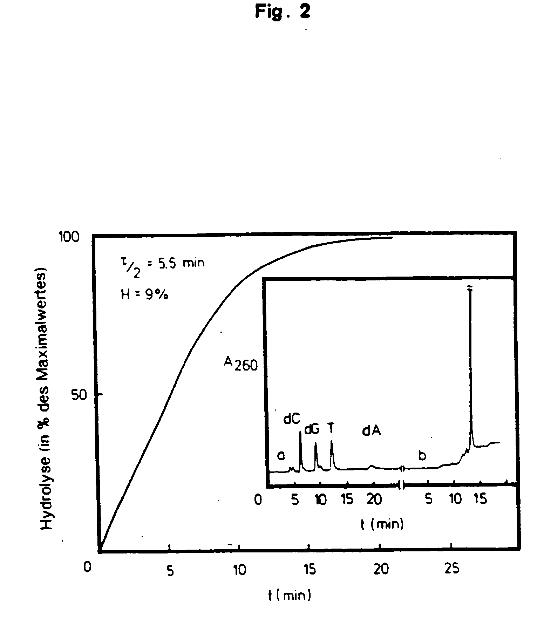

### Phosphodiesterase-Spaltung des Oligomers d(GTAGAAxTxTCTAC) (Fig. 2)

0,2 A₂₆₀ Einheiten des Oligonukleotides wurden in 200 µl 0,1 M Tris-HCl-Puffer (pH 8,3) gelöst und während 45 Min. bei 37°C mit 12 µg Kälbermilz-Phosphodiesterase (Boehringer Mannheim, Katalog Nr. 108 251) und anschließend während 30 Min. bei 37°C mit 2 µg Alkalischer Phosphatase (Boehringer Mannheim, Katalog Nr. 108 154) behandelt.

Die Reaktionsmischung wurde danach per HPLC analysiert (RP-18, Elutionsmittel 0,1 M (Et₃NH)OAc (pH 7)/Acetonitril (95:5) [A], gefolgt von 0-20 % Acetonitril [B] in [A], Flußrate ca. 1 ml/Min.).

Es zeigt sich, daß das Oligomer nur teilweise hydrolysiert wird; danach spaltet die Phosphodiesterase nur die nichtmodifizierten 5'-terminalen Nucleotide ab mit einem t/2 Wert von 5,5 Min.(Hypochromizität des Endprodukts 9 %, T_{M}=36°C). Wie das HPLC-Profil (eingesetzte Figur) zeigt, liegt danach der Großteil des Oligonukleotides in unveränderter Form vor.

### Beispiel 12 b

### Phosphodiesterase-Spaltung des oligomers d(GxTxAGAATTCxTxAC)

In analoger Weise wie unter Beispiel 12 a aufgeführt, wurde die Spaltung dieses Oligonucleotides mit Kälbermilz-Phosphodiesterase untersucht. Dabei wird nur noch eine sehr langsame und geringfügige Hydrolyse beobachtet (Hypochromizität des Endprodukts 3 %, T_{M} = 27 ° C).

### Beispiel 13

### Hybridisierungskomplex d(xT₁₂) mit d(A₁₂)

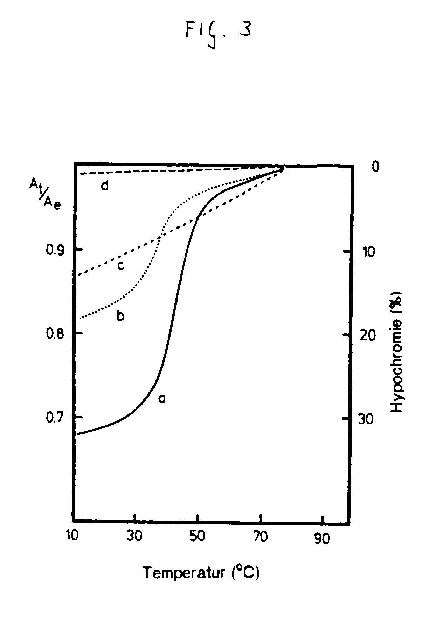

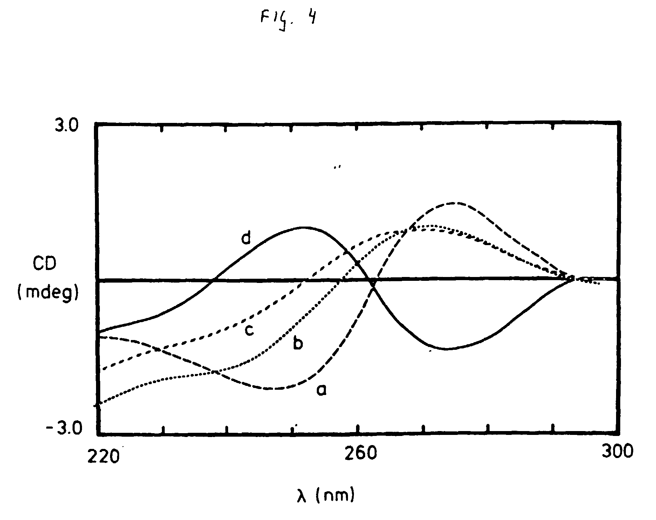

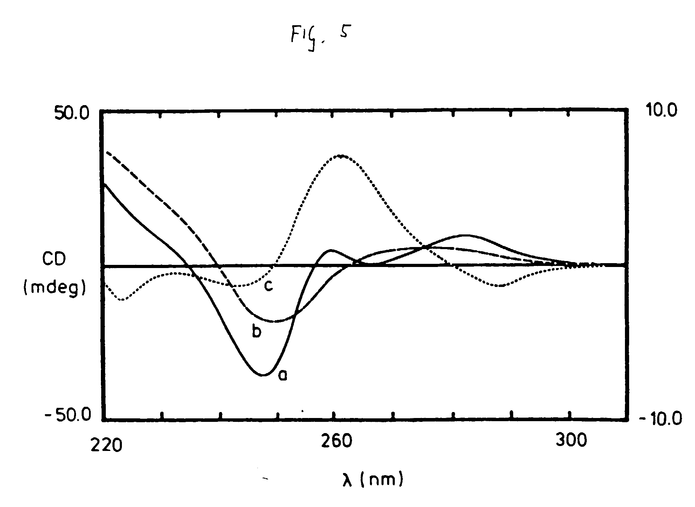

a) Schmelzkurven
   Hierzu wurden je 2 µMol der Oligonukleotide eingesetzt. Die Messung erfolgte in einer thermostatisch kontrollierten Zelle mit einem Shimadzu-210-A UV-Spektrophotometer in Verbindung mit einem Kipp und Zonen BD 90 Recorder. Die Zunahme der UV-Absorption bei entweder 260 oder 284 nm als Funktion der Zeit wurde aufgezeichnet, während die Temperatur der Lösung linear (10-80°C) mit 20°/Stunde erhöht wurde (Lauda PM-350 Programmierer in Verbindung mit einem Lauda RCS 6 Bad mit R 22 Einheit). Die jeweilige Temperatur wurde in der Referenz-Zelle mit einem Pt-Resistor gemessen. Die Hypochromie-Werte wurden aus der Anfangs- und Endabsorption errechnet. Die so erhaltenen Schmelzkurven sind in Fig. 3 dargestellt. Sie zeigen für das Hybrid d(A₁₂) / d (xT₁₂) (b) den für ein kooperatives Schmelzen von Doppelsträngen charakteristischen Verlauf (vgl. Kurve (a) für d(A₁₂) / d (T₁₂)), den die einzelsträngigen Homopolymere (dCA₁₂) (c) und d(T₁₂) (d)) nicht aufweisen.
b) CD-Spektroskopie (Fig. 3)
   Je 2 µmol der jeweiligen Einzelstränge (dA₁₂) und d(T₁₂) bzw. d(xT₁₂) wurden hybridisiert (60 mM Kakodylat-Puffer pH 7,0, 1 M NaCl, 100 mM MgCl₂, 8°C). Von den erhaltenen Hybriden sowie den Einzelsträngen d(T₁₂), d(xT₁₂) und d(A₁₂) und den Monomeren T und xT wurden an einem Jasco-600 Spektropolarimeter mit thermisch kontrollierter (Lauda RCS 6) 1 cm Küvette ein CD-Spektrum aufgenommen. Die erhaltenen Spektren sind in den Figuren 4 und 5 gezeigt.
   Wie ersichtlich, unterscheiden sich die CD-Spektren der Hybride deutlich von den Summen bzw. Differenzen der Einzelstränge. Dies zeigt, daß sich eine neuartige doppelsträngige Hybridstruktur ausbildet.

### Beispiel 14

### Bestimmung der viralen Gen Expression von HIV-I infizierten H 9 Zellen

Chronisch infizierte H 9 (ATCC CRL 8543) Zellen wurden in Gegenwart verschiedener Konzentrationen des Oligomers d(xTCG xTCG CxTG xTCxT CCG CxTxT CxTxT CCxT GCC xA) aus Beispiel 12 in 200 µl Kulturmedium (RPMI 1640 mit 15 % fötalem Kälberserum, 4 mmol L-Glutamin, 50 nmol 2-Mercaptoethanol, 50 Einheiten Penicillin und 50 µg Streptomycin/ml) kultiviert. Nach 6 Tagen Kultur wurden 100 µl des Kulturüberstandes entnommen und die Menge des p24 gag Proteins mittels Radioimmunoassay bestimmt.

Während im Kontrollexperiment (ohne Oligomer) die p24 gag Protein-Menge innerhalb von 5 Tagen auf Werte um 200 ng/ml anstieg, lag die Protein-Synthese unter Einsatz des Oligomeren Dosis-abhängig bei Werten von 50 ng/ml bis ca. 5 ng/ml.

### Beispiel 15

### 2.6 Diamino-9-(β-D-ribofuranosyl)purin (2) [1]

5.66 g (20 mmol) Guanosin (1) (getr. bei 80 C/P₂O₅) werden durch 10 stündiges Erhitzen auf 140 C mit 200 ml Hexamethyldisilazan (HMDS) und 0.5 ml Trimethylchlorsilan (TCS) silyliert; das überschüssige HMDS wird anschließend bei Normaldruck abdestilliert und der zurückgebliebene zähe Sirup mit einem Gemisch von 35 ml absolutem Toluol und 2 ml HMDS in einen 100 ml Autoklaven überführt. Nach Zusatz von 4 ml einer 0.5 M Lösung (2 mmol) von Trimethyl-silyltriflat ((CH₃)SiSO₃CF₃) in Benzol werden bei +5°C 30 min 5 atm NH₃ aufgepreßt; dann wird der Autoklav 48 h auf 150°C erhitzt. Nach vorsichtigem Ablassen des Ammoniaks wird der Inhalt des Autoklaven mit 150 ml Methanol herausgespült und nach Zusatz von 150 ml Wasser 4 h auf dem Dampfbad erhitzt. Nach Entfernen des Methanols verdünnt man mit Wasser auf 250 ml, entfärbt die siedende Lösung mit wenig Aktivkohle und filtriert, wobei man mit 100 ml heißem Wasser nachwäscht. Das gelbe Filtrat kristallisiert nach Einengen auf 250 ml langsam beim Abkühlen und Stehen bei 24°C. Man erhält nach Filtration und weiterem Einengen der Mutterlauge auf 100 ml 4.23 g (75.5%) farblose Kristalle von 2 [1]. DC (Kieselgel, Laufmittel EtOAc-MeOH; 60:40, Rf = 0.55).

### Beispiel 15

### 2,6 Diamino-9-(2-O-p-toluolsulfonyl-β-D-ribofuranosyl)purin (3) [2]

2.40 g (2) und 2.2 g Dibutylzinnoxid werden in 200 ml MeOH suspendiert und für 2 h unter Rückfluß gekocht, wobei langsam eine klare Lösung entsteht. Nach Abkühlen auf Raumtemperatur und Zugabe von 18 ml Triethylamin werden langsam 24 g p-Toluolsulfonsäurechlorid hinzugegeben und anschließend weitere 15 min gerührt. Das überschüssige Lösungsmittel wird bei 40°C abgezogen und der Rückstand mit 200 ml Wasser aufgenommen. Nach Extraktion mit Ether (2 x 100 ml) wird die wäßrige Phase auf 100 mL eingeengt und über Nacht bei 0°C aufbewahrt. Das dabei ausgefallene Rohprodukt wird gesammelt und durch Säulenchromatographie (Kieselgel, Laufmittel B) gereinigt. Die Mutterlauge wird zur Trockene eingeengt, an Kieselgel adsorbiert und ebenfalls chromatographiert. Man erhält 2.85 g (77%) der Verbindung (3) [2]. - F.p. = 134°C ; DC (Kieselgel Laufmittel EtOAc-MeOH, 85:15) Rf = 0.45; 1H-NMR ([D6]DMSO): 7.82 (s, H-C(8)); 6.83 (s, NH2); 5.91 (d, J(H-C(1'),H-C(2')= 7.5 Hz; H-C(1')); 5.67(s, NH2); 5.41 (m, Hα-C(2')); 4.29 (m, H-C(3')); 4.01 (H-C(4')); 3.55 (m, CH2); 3.17 (d, Hβ-C(C2')); 2.29 (s, - CH3)

### Beispiel 16

### 2'-Amino-(2'-desoxy-β -D-threopentofuranosyl)adenin (4) [2].

Zu einer Lösung von (3) (1.4 g; 2.75 mmol) in 30 ml trockenem DMSO wird unter Argon-Atmosphäre eine 1M Lösung von LiEt₃BH in THF (34 ml) gegeben. Die resultierende farblose Lösung wird über Nacht bei RT gerührt und tropfenweise über einen Zeitraum von 30 min mit 7 ml Wasser versetzt. Anschließend wird das Lösungsmittel abgezogen und der Rückstand an einer Dowex 1x2 (OH⁻) Säule (4x30 cm) mit 500 ml Wasser entsalzt. Das Reaktionsprodukt kann nun mit einer MeOH/Wasser-Mischung (1:1) von der Säule eluiert und nach Eindampfen der entsprechenden Fraktionen gewonnen werden. Umkristallisation aus Wasser (100 ml) ergibt 760 mg (98%) reines (4) [3]. F.p. 175 C. 1H-NMR ([D6]DMSO): 7.95 (s, H-C(8)); 6.78 (s, NH2); 6.07 (d, H-C(1')); 5.81 (s, NH2); 4.65 (m, OH-C(5')); 4.30 (br, H-C(3')); 3.84 (m, H-C(4')); 3.72 (m, CH2-C(5')); 2.73 (m, Hα-C(2')); 2.23 (d, J(Hα-C(2'), Hβ-C(2')= -14.5 Hz; Hβ-C(2')).

### Beispiel 17

### (2'-Desoxy-β-D-threo-pentofuranosyl)guanin (5)

Verbindung (4) wird in 100 ml Wasser unter Erwärmen gelöst und nach Abkühlung auf ca. 30°C mit Adenosindesaminase (200 µl;) versetzt. Nachdem das Reaktionsgemisch bei 30°C über Nacht gerührt wurde, kontrolliert man den Reaktionsverlauf mittels DC und UV-Spektroskopie. Nach Abziehen des Lösungsmittels dampft man das Reaktionsprodukt mehrmals mit Aceton ab und erhält schließlich 650 mg (92%) (5). F.p. > 250 C; DC (Kieselgel, Laufmittel iPrOH-25%NH₃aq-H₂O; 3:1:1): Rf = 0.61. 1H-NMR ([D6]DMSO): 7.95 (s, H-C(8)); 6.49 (s, NH2); 6.05 (d, H-C(1')); 5.43 (s, OH-C(3')); 4.67 (s, OH-C(5')); 4.33 (d, H-C(3')); 3.87 (s, H-C(4')); 3.65 (m,CH2-C(5')); 2.70 (m, Hα-C(2')); 2.20 (d, Hβ-C(2')).

### Beispiel 18

### N2,3',5'-Triisobutyryl-(2'-desoxy-β-D-threo-pentofuranosyl)guanin (6)

500 mg (5) werden durch mehrmaliges Abdampfen mit absolutem Pyridin getrocknet und in 20 ml Pyridin unter leichtem Erwärmen gelöst. Nach Abkühlen auf 0°C im Eisbad werden unter Argon 1.95 ml (18.5 mmol) Isobuttersäurechlorid langsam unter kräftigem Rühren gegeben. Die trübe, gallertartige Reaktionsmischung wird 1 h bei RT weitergerührt und anschließend mit einer Lösung von 2.5 g NaHCO₃ in 40 ml H₂O hydrolysiert. Nach Einengen der Lösung auf ca. 20 ml und Stehenlassen bei 0°C über Nacht wird das Reaktionsprodukt abgesaugt und mit 20 ml Ether nachgewaschen. Nach Säulenchromatographie mit Kieselgel (5 cm x 30 cm), Laufmittel CHCl₃-MeOH; 8:2, erhält man 820 mg amorphes (6) [4]. F.p. = 91 C, DC (Laufmittel CHCl₃-MeOH; 8:2) Rf = 0.40; 1H-NMR ([D6]DMSO): 12.05 (br, NH); 11.70 (br, NH); 8.14 (s, H-C(8)); 6.14 (d, H-C(1')); 5.30 (s, OH-C(3')); 4.68 (s, OH-C(5'); 4,36 (s, H-C(3')); 3.94 (m, H-C(4')); 3.72 (m, CH2-C(5')); 2.77 (m, Hα-C(2')); 2.28 (d,Hβ-C(2')); 1.13 (m, 7H-iBut.); C₂₂H₃₁N₅O₇ (477.51): ber.: C 55.34, H 6.54, N 14.67; gef.: C 55.18, H 6.57, N 14.73.

### Beispiel 19

### N2-Isobutyryl-(2'-Desoxy-β-D-threo-pentofuranosyl)guanin (7)

1.2 g (2.5 mmol) (6) werden in 40 ml MeOH gelöst und im Eisbad auf 0°C gekühlt. Anschließend wird 1N wässrige NaOH-Lsg. portionsweise zugegeben, bis der pH-Wert auf 12 angestiegen ist. Nach 50 min wird die Reaktion durch Zugabe von Ionenaustauscher (Dowex W x 8; Pyridinium-Form) gestoppt und die neutrale Lösung abfiltriert. Nach Waschen des Harzes mit MeOH wird das Lösungsmittel abgezogen und der Rückstand aus sehr wenig Wasser umkristallisiert. Nach Vervollständigung der Kristallisation durch Stehenlassen über Nacht im Kühlschrank erhält man 560 mg (72%) weiße Nadeln der Verbindung (7). F.p. > 250 C; DC (Kieselgel, Laufmittel CHCl₃-MeOH, 9:1) Rf = 0.42; 1H-NMR ([D6]DMSO): 12.05 (br, NH); 11.70 (br, NH); 8.20 (s, H-C(8)); 6.14 (d, H-C(1')); 5.30 (s, OH-C(3')); 4.68(s, OH-C(5')); 4.36 (s, H-C(3')); 3.94 (m, H-C(4')); 3.72(m, CH2-C(5')); 2.77 (m, Hα-C(2')); 2.28 (d, Hβ-C(2')); 1.13 (m, 7H-i-But.); C₁₄H₁₉N₅O₅ (337.3): C 49.85, H 5.68, N 20.76; gef.: C 49.98, H 5.81, N 20.75.

### Beispiel 20

### N2-Isobutyryl-5'-O-dimethoxytrityl-(2'-desoxy-β-D-threo-pentofuranosyl)guanin (8).

300 mg (7) (0.89 mmol) werden durch mehrmaliges Abdampfen mit abs. Pyridin getrocknet und anschließend in 5 ml abs. Pyridin gelöst. Nach Zugabe von Dimethylaminopyridin (16 mg, 0.13 mmol) werden unter Argon 150 mg (0.4 mmol) 4',4'-Dimethoxytritylchlorid hinzugegeben. Nach 4 h weiterem Rühren bei RT und anschließender Zugabe von 45 mL 5%iger NaHCO₃-Lsg. wird mit CH₂Cl₂ extrahiert (3 x 50 mL). Die vereinigten org. Phasen werden über Na₂SO₄ getrocknet, filtriert und die Lösungsmittel abgezogen. Nach Chromatographie an Kieselgel (20 x 5 cm, Laufmittel CHCl₃-MeOH, 8:2) und Eindampfen der Hauptzone erhält man 423 mg (74%) amorphes (8). DC (Kieselgel, Laufmittel CHCl3-MeOH, 8:2) Rf = 0.38.
1H-NMR ([D6]DMSO): 12.09 (br, NH); 11.77 (br, NH); 8.07 (s, H-C(8)); 6.22 (d, H-C(1')); 5.30 (s, OH-C(3')); 4.34 (s, H-C(3')); 4.21 (s, H-C(4')); 3.71 (s, 3H-OCH3); 3.20 (m,H-C(5')); 2.74 (m, Hα-C(2')); 2.30 (m, Hβ-C(2')); 1.12 (m, 7H-i-But.);

### Beispiel 21

### N2-Isobutyryl-5'-dimethoxytrityl-(2'-desoxy-β-D-threo-pentofuranosyl) guanin 3'-Phosphonat (9a)

Die Verbindung wurde wie für Verbindung 16 beschrieben dargestellt und aufgearbeitet.

### Beispiel 22

### N2Isobutyryl-5'-dimethoxytrityl-(2'-desoxy-β-D-threo-pentofuranosyl)guanin 3'[(2-Cyanoethyl)-N,N-Diisopropylphosphoramidit] [9b]

Die Verbindung 9b wurde wie in [6] beschrieben dargestellt und aufgearbeitet.

### Beispiel 23

### 9-(2'-Desoxy-β-D-threo-pentofuranosyl)guanin 3'-[3-(N-'Fractosil'carbamoyl)propanoat] [9c]

Die Verbindung 9c wurde wie in [6] beschrieben dargestellt und aufgearbeitet.

### Beispiel 24

### 4-Benzoylamino-1-(2'-desoxy-β-D-erythro-pentofuranosyl)-2(1H)-pyrimid inon (10)

Verbindung 10 wurde dargestellt wie bei Ti et al. beschrieben.

### Beispiel 25

### O2,3'-Anhydro-1-[2'-desoxy-5'-O-(4-methoxybenzoyl)-β-D-threopentofur anosyl)-4-benzoylcytosin (11) [3]

Zu einer Lösung von (10) (3.3 g, 10 mmol) und PPh₃ (4 g, 15 mmol) in trockenem DMF (20 ml) wird eine Lösung von Diisopropylazodicarboxylat (DIAD, 3 ml, 15 mmol) und p-Methoxybenzoesäure (2.3 g, 15 mmol) in trockenem DMF (11 ml) innerhalb 5 min zugetropft. Die Reaktionsmischung läßt man 15 min bei RT rühren und versetzt anschließend mit der gleichen Menge Triphenylphosphin und DIAD. Man läßt weitere 30 min bei RT rühren. Anschließend gießt man die Lösung in eisgekühlten Et₂O (250 ml) und kühlt die erhaltene Suspension 2 h. Der weiße Rückstand wird abfiltriert und mit Et₂O gewaschen. Durch Umkristallisation aus Ethanol erhält man Verbindung (11) als farblose Plättchen (3.42 g, 76%), Schmp. 188 C, Rf (CH₂Cl₂/CH₃OH): 0.38. UV (MeOH): lmax (ε) = 318, 254 (30440, 17150). 1H-NMR [(d6)-DMSO]: 2.59-2.71 (m, Jgem =12.5 Hz, 2H, H-2'β, H-2'α); 4.41 (m, 2H, H-5'); 4.58 (m, 1H, H-4'); 5.47 (m, 1H, H-3'); 6.00 (m, 1H, H-1'); 6.52 (d, J5,6 = 7.25 Hz, 1H, H-5); 7.01, 7.84 (AB, q, 4H, C6H4); 7.68 (d, 1H, H-6); 7.45, 7.95 (m, 5H, arom. H). C₂₄H₂₁N₃O₇ (447.4): ber.: C 64.44, H 4.73, N 9.39; gef.: C 64.49, H 4.71, N 9.31.

### Beispiel 26

### 4-Amino-1-(2'-Deoxy-β-D-threo-pentofuranosyl)-2(1H)-pyrimidinon (12)

Eine Lösung von (11) (2.0 g, 4.5 mmol) gelöst in Ethanol/Wasser (1:1, 250 ml) wird mit Dowex Ionenaustauscher versetzt (OH--form; 250 ml, in Wasser suspendiert). Man läßt 16 h bei 50°C rühren. Der Ionenaustauscher wird abfiltriert und intensiv mit Wasser gewaschen. Filtrat und Schlamm werden eingedampft, der ölige Rückstand in wenig Methanol/Ethylacetat (1:1, 50 ml) aufgenommen und im Ölpumpenvakuum eingeengt. Man erhält Verbindung (12) als einen farblosen Schaum (965 mg, 95%). Die UV-Daten der Verbindung (12) waren identisch mit den Daten von (11). 1H-NMR [(d6)-DMSO]: 1.82 (m, J2'β,2'α = 14.5 Hz, H-2'β); 2.57 (m, 1H, H-2'α); 3.68 (m, 2H, H-5'); 3.81 (m, 1H, H-4'); 4.23 (m, 1H, H-3'); 4.73 (br s, 1H, 5'-OH); 5.19 (br s, 1H, 3'-OH); 5.73 (d, J5,6 - 7.25 Hz, 1H, H-5); 6.02 (m, 1H, H-1'); 7.11 (br s, 2H, NH2); 7.84 (d, 1H, H-6).

### Beispiel 27

### 1-(4-Benzoylamino-2'-desoxy-β-D-threo-pentofuranosyl]cytosin (13) [4]

Nucleosid 12 (454 mg, 2 mmol), das zuvor dreimal mit trockenem Pyridin durch Abdampfen getrocknet wurde, wird in abs. Pyridin (10 ml) suspendiert und unter Argon mit (CH₃)₃SiCl (1.28 ml, 10 mmol) versetzt. Die Lösung wird 15 Minuten gerührt, danach wird Benzoylchlorid (0.87 ml, 10 mmol) hinzugefügt und die Lösung 2 h bei RT gerührt. Anschließend wird die Mischung im Eisbad abgekühlt und mit Wasser (2 ml) versetzt. Nach der Zugabe von 25%iger Ammoniak-Lösung (2 ml) wird weitere 15 Minuten bei RT gerührt. Die Lösung wird fast bis zur Trockne eingedampft, der Rückstand in Wasser (28 ml) gelöst und mit Essigsäureethylester (10 ml) gewaschen. Nach dem Einengen der wässrigen Phase beginnt das Produkt (13) unter Kühlung auszukristallisieren. Durch Umkristallisation aus Methanol erhält man weiße Nadeln. (450 mg, 68%) Schmp.: 177 C (MeOH). DC (CH₂Cl₂/CH₃OH 9:1): Rf 0.36. UV (MeOH): max (ε) = 302, 258 (10500, 22230). C₁₆H₁₇N₃O₅ (331.70): ber.: C 58.01, H 5.17, N 12.68; gef.: C 58.14, H 5.29, N 12.71.
1H-NMR [(d6)-DMSO]: 2.01(d, JH-2'β, H-2'α =14.5 Hz, 1H, H-2'β,); 2.57(m, 1H, H-2'α); 3.78 (m, 2H, H-5', H-5"); 3.98 (m, 1H, H-4'); 4.35 (m, 1H, H-3'); 4.76 (t,J5'-OH, H-5', H-5" = 5.5 Hz, 1H, 5'-OH); 5.09 (d, J3'-OH, H-3'= 2.75, 1H, 3'-OH);6.00 (dd,
J = 7.25 Hz, 1H, H-1'); 7.31 - 8.02 (m, 5H, arom.H); 7.63 (d, JH-5,H-6= 7.5 Hz, 1H, H-6); 8.28 (d, JH-6,H-5= 7.5 Hz, 1H, H-6); 11.19 (brs, 1H, NH).

### Beispiel 28

### 1-[5'-O,4-N-Bis(4,4'-dimethoxytrityl)-2'-desoxy-β-D-threo-pentofuranosyl]cytosin (14a)

Trockenes (12) (520 mg, 2,2 mmol), das durch Abdampfen mit trockenem Pyridin erhalten werden kann, wird in abs. Pyridin (10 ml) gelöst. Zu dieser Lösung wird 4,4'-Dimethoxytritylchlorid (1.5 g, 4 mmol) und 4-Dimethylaminopyridin (150 mg, 1.26 mmol) hinzugefügt.

Nach fünf Stunden zeigt das DC fast kein Ausgangsmaterial mehr an. Die Reaktionsmischung wird in kalte, gesättigte Natriumhydrogencarbonat-Lsg. (50ml) gegossen und mit drei Portionen Ethylacetat (30 ml) extrahiert. Die gesammelten organischen Phasen werden eingedampft und der Rückstand durch Säulenchromatographie gereinigt (Säule 6 x 30 cm, 0.1 bar,
CH₂Cl₂/MeOH/Et₃N erst 93:5:2, dann 88:10:2).
Die geeigneten Fraktionen der unpolaren Substanz (14a) werden gesammelt, das Lösungsmittel abgedampft, der Rückstand in wenig Dichlormethan gelöst und tropfenweise unter Rühren zu Petrolether gegeben, wodurch (14a) als weißer Feststoff ausfällt (680 mg, 40%).
DC(CH₂Cl₂/CH₃OH 9:1): Rf 0.46. UV (MeOH): max (ε) = 280, 230 (18100, 43000). 1H-NMR [CDCl3]: 2.17(d, JH-2'β, H-2'α =14.3 Hz, 1H, H-2'β,); 2.52(m, 1H, H-2'α); 3.46 (m, 2H, H-5', H-5"); 3.74(s, 12H, OCH3); 3.96 (m, 1H, H-4'); 4.35 (m, 1H, H-3'); 4.99 (d, JH-5,H-6 = 7.75 Hz, 1H, H-5); 6.01 (dd,J = 6.25 Hz, 1H, H-1'); 6.75 - 7.38 (m, 26H, arom.H); 7.51 (d, JH-5,H-6= 7.75 Hz, 1H, H-6). C₅₁H₄₉N₃O₈ (831.96): ber.: C 73.63, H 5.94,N 5.05; gef.: C 73.62, H 6.08,N 5.06.

### Beispiel 29

### 1-[5'-O-(4,4'-Dimethoxytrityl)-2'-desoxy-β-D-threo-pentofuranosyl] cytosin (14b) [2]

Die Fraktionen der polaren Substanz (14b) werden ebenfalls gesammelt, das Lösungsmittel abgedampft, der Rückstand in wenig Dichlormethan aufgenommen und tropfenweise unter Rühren zu Petrolether gegeben, wodurch (14b) als weißer Feststoff ausfällt. (580 mg, 55%). DC (CH₂Cl₂/CH₃OH 9:1): Rf 0.36. UV (MeOH): max (ε) = 234, 274 (22200, 8970). 1H-NMR [(d6)-DMSO]: 1.80(d, JH-2'β, H-2'α =14.5 Hz, 1H, H-2'β,); 2.50*)(m, 1H, H-2'α); 3.18 (m, 2H, H-5', H-5"); 3.74(s, 6H, OCH3); 4.06 (m, 1H, H-4'); 4.15 (m, 1H, H-3'); 5.12 (d, J3'-OH,H-3' = 3.70 Hz, 1H, 3'-OH); 5.64 (d, JH-5,H-6=7.43 Hz, 1H, H-1'),6.04 (dd,J = 6.5 Hz, 1H, H-1'); 6.87 - 7.44 (m, 15H, arom.H); 7.66 (d, JH-5,H-6= 7.43 Hz, 1H, H-6). C₃0H₃₁N₃O₆ (529.59): C 68.04, H 5.90, N 7.93; gef.: C 68.03, H 5.93, N 7.89.

### Beispiel 30

### 1-[4-Benzoylamino-2'-desoxy-5'-O-(4,4'-Dimethoxytrityl)-β-D-threo-pentofuranosyl] cytosin (15)

Verfahren A [8]: Verbindung 13 (270 mg, 0.8 mmol) wird durch Abdampfen mit trockenem Pyridin getrocknet. Der Rückstand wird in abs. Pyridin (10 ml) gelöst. Unter Argon wird DMT-Cl (0.6 g; 1.8 mmol) und 4-Dimethylaminopyridin (200 mg; 1.8 mmol) hinzugegeben und die Lösung 4 Stunden bei Raumtemperatur gerührt. Die Lsg. wird in 5%ige NaHCO₃-Lsg. (27 ml) geschüttet, zweimal mit Dichlormethan (50 ml) extrahiert, die gesammelten organischen Phasen über Na₂SO₄ getrocknet und das Lösungsmittel abgedampft. Nach wiederholtem Abdampfen mit Toluol wird der Rückstand durch Flashchromatographie gereinigt (Säule 6x20 cm, 0.1 bar, CH₂Cl₂/MeOH/Et₃N 93:5:2). Das Produkt (15) wird als weißes Pulver aus Petrolether gefällt. (330 mg, 64%)

Verfahren B: Das Reaktionsgemisch von (14a) und (14b) wird nach der Aufarbeitung (ohne Säulenchromatographie) in abs. Pyridin (10 ml) gelöst. Unter Argon wird die Lösung mit (CH₃)₃SiCl (1,28 ml, 10 mmol) versetzt. Nach 15 Minuten wird Benzoylchlorid (1.16 ml, 10 mmol) hinzugegeben und das Reaktionsgemisch drei Stunden bei RT gerührt. Nach Abkühlung auf 0 C wird Wasser (2 ml) und nach weiteren fünf Minuten 25%ige Ammoniak-Lösung (4 ml) hinzugegeben. Nach 30 minütigem Rühren bei RT wird die Lösung eingedampft und der gummiartige Rückstand in Dichlormethan (20 ml) und 5%iger NaHCO₃-Lsg. (40 ml) extrahiert. Die wässrige Phase wird mit 2 Portionen Dichlormethan extrahiert, die organischen Phasen kombiniert und das Lösungsmittel abgedampft. Die Isolierung erfolgt in gleicher Weise wie oben beschrieben. (530 mg, 36.5%)
DC (CH₂Cl₂/MeOH 9:1): Rf 0.54. UV (MeOH): max (ε) = 301, 256, 236 (14540, 28880, 37040). 1H-NMR [(d6)-DMSO]: 2.00(d, JH-2'β, H-2'α =14.5 Hz, 1H, H-2'β,); 2.50*)(m, 1H, H-2'α); 3.39 (m, 2H, H-5', H-5"); 3.75 (s, 6H, OCH3); 4.23 (m, 2H, H-4', H-3'); 5.07 (d,J3'-OH,H-3' = 2.50 Hz, 1H, 3'-OH); ; 6.05 (dd,J = 7 Hz, 1H, H-1'); 6.89 - 7.62 (m, 18H, arom.H); 7.53 (d, JH-5,H-6=7.43 Hz, 1H, H-1'); 8.00 (d, JH-5,H-6= 7.43 Hz, 1H, H-6); 11.20 (brs, 1H, NH). C₃₇H₃₅N₃O₇ (633.70): C 70.13, H 5.57, N 6.63; gef.: C 70.15, H 5.64, N 6.46.

### Beispiel 31

### 1-[4-Benzoyl-2'-desoxy-5'-O-(4,4'-dimethoxytrityl)-β-D-threopentofuranosyl]cytosin-(3'-hydrogen-phosphonat) (16)

Zu einer Lösung von N-Methylmorpholin (6.5 ml; 57.5 mmol) in 23 ml absolutem Dichlormethan werden unter Argon PC13 (500 µl; 5.75 mmol) und 1,2,4-Triazol (1.35 g; 19.4 mmol) gegeben. Die Lösung wird 30 Minuten gerührt, danach auf 0 C abgekühlt und mit (15) (400 mg; 0.63 mmol) versetzt, das zuvor durch Abdampfen mit absolutem Acetonitril getrocknet wurde, in 7 ml abs. Dichlormethan gelöst und langsam bei RT zugetropft. Nach 20 Minuten Rühren bei RT gießt man die Lösung in 32 ml TBK-Puffer (pH 7.5-8.0) und trennt die Phasen. Die wässrige Phase wird 4 mal mit Dichlormethan (15 ml) extrahiert, die vereinigten. organischen Phasen über Na₂SO₄ getrocknet, filtriert und das Lösungsmittel abgedampft. Der hellgelbe Schaum wird chromatographiert (Kieselgelsäule 6 x20 cm, 0.5 bar, 1 Liter CH₂Cl₂/Et₃N 98:2, dann CH₂Cl₂/MeOH/Et₃N 88:10:2). Der Rückstand der Hauptzone wird in CH₂Cl₂ gelöst, mit 0.1 M TBK-Puffer (10 ml) zehnmal ausgeschüttelt, die wässrige Phase mehrmals mit CH₂Cl₂ zurückgeschüttelt, die gesammelten organischen Phasen über Na₂SO₄ getrocknet und das Lösungsmittel abgedampft. Das H-Phophonat liegt als farbloser Schaum vor. (260 mg; 51%).
DC (CH₂Cl₂/MeOH/Et₃N 88:10:2): Rf 0.35. 1H-NMR [CDCl3]: 1.18 (t, J = 7.25 Hz, 9H, 3xCH3CH2NH); 2.47 (d, J H-2'α, H-2'β = - 15Hz, 1H, H-2'β); 2.62 (m, 1H, H-2'α); 2.90 (q, J = 7.25 Hz, 6H, 3xCH3CH2NH); 3.55 (m, 2H, 5'-CH2); 3.77 (s, 6H, OCH3); 4.27 (m, 1H, H-4'); 4.78 (m, 1H, H-3'); 5.31,7.81 (d, JH-P = 625 Hz, 1H, H-P); 6.16 ("dd", J = 6.75 Hz, 1H, H-1'); 6.80 - 7.90 (m, 18H, arom H); 7.21 (d, JH-5,H-6 = 7.25 Hz, 1H, H-5); 8.08 (JH-6,H-5 - 7.25 Hz, 1H, H-6). 31P-NMR [d6-DMSO]: 0.60 1J(P,H = 590 Hz; 3J(P,H-C4' = 8.4 Hz). 31P-NMR [CDCl3]: 3.30 1J(P,H = 625 Hz; 3J(P,H-C4' = 8.9 Hz).

### Beispiel 32

### 1-[4-Benzoyl-2'-desoxy-5'-O-dimethoxytrityl-β-D-threo-pentofuranosyl] cytosin 3'-[(2-Cyanoethyl)-N,N-diisopropylphosphoramidit] [16b].

Verbindung 16b wurde wie in [6] beschrieben dargestellt und aufgearbeitet.

### Beispiel 33

### 1-[2'-Desoxy-β-D-threo-pentofuranosyl)cytosin 3'-[3-(N-'Fractosil'carbamoyl)propanoat] [16c]

Verbindung 16c wurde wie in [6] beschrieben dargestellt und aufgearbeitet.

### Literaturverzeichnis zu den Beispielen 15 - 33

[1] H. Vorbrüggen, K Krolikiewicz, Liebigs Ann. Chem. 1976, 745.
[2] M. J. Robins, S. G. Wood, N. K. Dalley, P. Herdewijn, J. Balzarini, E. deClercq, J. Med. Chem. 1989, 32, 1763.
[3] S. Czernecki, J. M. Valery, Synthesis 1991, 239.
[4] G. S. Ti, B.L. Gaffney, R. A. Jones, J. Amer. Chem. Soc. 1982, 104, 1315.
[5] B. C. Froehler, P.G. Ng, M. D. Mateucci Nucleic Acids Res. 1986, 14, 5399.
[6] H. Rosemeyer, F. Seela, Helv. Chim. Acta 1991, 74, 748.

### Beispiel 34

### Hemmung der HIV-Virus-Expression durch erfindungsgemäße Oligonucleotide

5'- xTxTT CCC AGG CTC AGA TCT GGT CxTxT T*- 3'                    (tar Region)

5'- xTxTT CGT CGC TGT CTC CGC TTC TTC CTG CCA xTxTT*- 3'      (rev Region)

5'- xTxTC TGC TAG AGA TTT TCC ACA CxTxT T*- 3'                    (primer binding
                                                                                                    site)
* aus Gründen der kommerziellen Erhältlichkeit des CPG-Trägermaterials
  wurde von unmodifiziertem 2'-Desoxy-ribo-thymidin ausgegangen

Virus HIV III B H 9
Stammlösungen der Oligomeren: 1 mg x ml⁻¹ in PBS-Puffer
Verdünnungsreihe der Oligomeren: 0,05 - 50 ug x ml⁻¹
Testsystem I : MT-2 (T-lymphoblastoid cells)
Testsystem II: PBMC (peripher blood monocytere cells)

### Testdurchführung:

In Mikrotiterplatten wurden zu je 100 ul der jeweiligen Oligonucleotidlösung 2 x 10⁴ bzw. 2 x 10⁵ infizierte MT-2-bzw. PBMC - Zellen gegeben.

Nach 7-tägiger Inkubation bei 37°C wurde auf Syncytien-Bildung kontrolliert und die Reduktion des cytopatischen Effektes über einen Farbtest mit MTT bzw. p24 - ELISA gemessen. Die Ergebnisse zeigt Tabelle I.

**Tabelle I**

| Oligonucleotid Region | Konzentration ug/ml | CPE -Reduktion % |
|---|---|---|
| tar | 0,05 | 59,1 |
| | | |
| rev | 0,05 | 40,7 |
| pbs | 0,05 | 42,2 |

Mit den in den Beispielen beschriebenen Monomeren können beliebige erfindungsgemäße Oligonukleotide (z. B. analog Beispiel 11 und 22) synthetisiert werden.

### Beispiel 35

### 1-(2-Deoxy-β-D-threo-pentofuranosyl)thymine Cyclic 3',5'-Monophosphate, Triethylammonium (2).

Allgemeines zur Herstellung von Desoxyxylonukleosid-5'-mono, di- und triphosphaten

Zur Herstellung der 5'-Mono-, Di- oder Triphosphate wird zuerst die 3'-OH-Gruppe benzoyliert und dann mit POCl₃ in Trialkylphosphat, vorzugsweise Trimethylphosphat oder Triethylphosphat, zum 5'-Phosphat phosphoryliert.
Anschließend wird die 3'-Schutzgruppe mit einer Base, beispielsweise Ammoniak, entfernt, wobei ggf. auch eine entsprechende Schutzgruppe, die bei einigen Nucleotiden in die heterocyclische Base eingeführt wurde, gleichzeitig mitabgespalten wird. Eine entsprechende
Schutzgruppenstrategie ist auch zur Herstellung der Thiophosphate verwendbar.

Eine Lösung von
(1-(2-deoxy-β-D-*threo*-pentofuranosyl)thymine (1) (59 mg, 0,24 mmol) in Trimethylphosphat (1 ml) wird mit POCl₃ (40 µl, 0,42 mmol) bei 4°C für fünf Stunden inkubiert.

Es wird Triethylammoniumbicarbonat (1 mol, pH 7,6, 10 ml) zugegeben und das Reaktionsgemisch nach einer Stunde Rühren bei Raumtemperatur eingedampft.

Der Rest wird an DEAE Sephadex (HCO₃⁻Form, Kolonne 30 x 2 cm) chromatographiert. Die Chromatographie wird mit 400 ml Wasser und anschließend einem linearen Gradienten von
Triethylammoniumbicarbonat (0 - 0,3 mol/l, 1200 ml) durchgeführt. Das Cyclophosphat eluiert bei 0,2 mol/l. Die Fraktionen, die das Produkt enthalten, werden eingedampft, dreimal in 50 ml Wasser aufgenommen und wieder eingedampft.
Das Produkt wird nochmals an DEAE Sephadex unter den gleichen Bedingungen chromatographiert. Die verdampfbaren Salze werden durch wiederholte Coverdampfung mit Wasser, Ethanol und Aceton entfernt.
1394 A₂₆₇ units (65%) of a colourless solid. TLC (2-propanol/25% aq. amnonia/water, 7:1:1): R_{f} 0.45; (EtOAc/acetone/EtOH/water, 15:3:4:3): R_{f} 0.09. HPLC (5% MeCN in 0.1 M triethylammonium acetate, pH 7.0; 0.6 ml/min): t_{R} 14.9 min.
Electrophoresis: E_{UP} 0.64. UV (MeOH): ₘₐₓ 267 nm. ³¹P-NMR (D₂O/0.1 M Tris-HCl buffer, 1:1): - 5.03 (d, J = 17.3). ¹H-NMR ((D₆)DMSO): 11.28 (s, NH); 10.79 (br, NH); 7.87 (s, H-C(6)); 6.12 (d, J = 7.9, H-C(1')); 4.69 (br, H-C(3')); 4.40 - 4.14 (m, CH₂ (5')); 3.64 (br, H-C(4')); 3.04 (q, CH₂ (Et)); 2.66 (m, H_{α}-C(2')); 1.96 (d, J = -16.2, H_{β}-C(2')); 1.76 (s, Me-C(5)); 1.20 (t, Ne-(Et)).

### Beispiel 36

### 9-(2-Deoxy-β-D-threo-pentofuranosyl)adenine Cyclic 3',5'-Monophosphate, Triethylammonium (4a).

### Verfahren A:

Die Reaktion von Verbindung 3
(9-(2-deoxy-β-D-*threo*-pentofuranosyl)adenine

(25 mg, 0,1 mmol) mit POCl₃ in Trimethylphosphat wird durchgeführt, wie für Verbindung 2 in Beispiel 35 beschrieben, jedoch in Gegenwart von 2-t-Buthylimino-2-diethyl-amino-1,3-dimethyl-perhydrodiazaphosphorin (BEMP) (41 mg, 0,15 mmol). Die Reaktion ist nach 12 Stunden abgeschlossen. Die Aufarbeitung erfolgt wie in Beispiel 35 beschrieben. Die Endreinigung nach Chromatographie an Sephadex wird über Dünnschichtchromatographie EtOAc/Aceton/EtOH/Wasser, 15:3:4:3 als Lösungssystem durchgeführt (R_{f} 0,13).

### Methode B:

Die Reaktion wird mit der Verbindung 3 (20 mg, 0,08 mmol) durchgeführt, wie für Methode A beschrieben, jedoch ohne Zugabe von BEMP. Nach Chromatographie an DEAE Sephadex Adenin (5 mg, 46 %) wird zunächst mit Wasser eluiert und dann die Verbindung 4 a) mit Puffer eluiert. Es wird eine farblose Substanz erhalten (567 A₂₆₀ units, 39 %).

### Methode C:

POCl₃ (20 µl, 0,21 mmol) wird zu einer Lösung von Imidazol (86 mg, 1,26 mmol) in MeCN (0,5 ml) zugegeben. Es wird 30 Minuten bei Raumtemperatur gerührt, auf 0°C abgekühlt und die Lösung von 9 b) (35 mg, 0,1 mmol) in MeCN/Dimethylformamid (1:1, 1 ml) zugegeben. Das Reaktionsgemisch wird für zwei Stunden bei 4°C und anschließend für 14 Stunden bei Raumtemperatur inkubiert. Nach Neutralisation mit 1 mol/l wässrigem Triethylammoniumbicarbonat (12 ml), wird die Lösung für 1 Stunde gerührt und eingedampft. Der Rest wird mit 25 %iger wäßriger Ammoniaklösung (20 ml) behandelt, für 16 Stunden, und eingedampft. Die Reinigung von Verbindung 4a) wird an DEAE Sephadex, wie beschrieben, durchgeführt. Das ungeschützte Nucleosid 3 wird nach Elution mit Wasser (24 %) erhalten, die Verbindung 4a) wird mit einer Lösung von 0,2 mol/l Triethylammoniumbicarbonat als farblose Verbindung erhalten (843 A₂₆₀ units, 57 %).

### Beispiel 37

### 1-[3-O-Benzoyl-2-deoxy-5-O-(4,4'-dimethoxytriphenylmethyl)-β-D-threo-pentofuranosyl]thymine (6a).

Benzoylcyanid (75 mg, 0,57 mmol) und Triethylamin (80 µl, 0,57 mmol) werden zu einer Lösung von Verbindung 5a) (265 mg, 0,49 mmol) in MeCN (4 ml) zugegeben. Es wird 1 Stunde bei Raumtemperatur gerührt, eingedampft und der Überrest an einer Silikagelsäule chromatographiert (2 x 17 cm) in CH₂Cl₂/Aceton, 6:1. Die Verbindung 6a) wird als weißer Schaum (255 mg, 81 %) erhalten.

TLC (CH₂Cl₂/acetone, 6:1): R_{f} 0.56. ¹H-NMR ((D₆)DMSO): 11.32 (s, NH); 7.72 - 7.17 (m, 14 arom. H and H-C(6)); 6.80 - 6.61 (m, 4 arom. H); 6.18 (d, J = 5.7, H-C(1')); 5.68 (m, H-C(3')); 4.48 (m, H-C(4')); 3.69 (m, CH₂(5') and 2s, 2 MeO); 2.88 (m, H_{α}-C(2')); 2.26 (d, J = - 16.2, H_{β}-C(2')); 1.55 (s, Me). Anal. calcd. for C₃₈H₃₆N₂O₈ (648.71): C 70.36, H 5.59 N 4.32; found: C 70.31, H 5.70, N 4.36.

### Beispiel 38

### 1-(3-O-Benzoyl-2-deoxy-β-D-threo-pentofuranosyl)thymine (7a).

Die Lösung von 6a) (200 mg, 0,31 mmol) in 80 % Essigsäure (3 ml) wird bei Raumtemperatur 15 Minuten gerührt, mit 100 ml Wasser verdünnt und eingedampft. Der Überrest wird je einmal mit 50 ml Wasser, 2-Propanol (50 ml) und 50 ml Methanol aufgenommen und eingedampft und an Silikagel (2 x 12 cm) in CH₂Cl₂/MeOH 95:5 chromatographiert, wobei Verbindung 7a) als weißer Schaum (98 mg, 92 %) erhalten wird.

TLC (CH₂Cl₂/MeOH, 95:5): R_{f} 0.30. ¹H-NMR ((D₆)DMSO): 11.29 (s, NH); 7.98 - 7.51 (m, 5 arom. H and H-C(6)); 6.14 (dd, J = 7.8, 2.3, H-C(1')); 5.54 (m, H-C(3')); 4.98 (t, OH-C(3')); 4.19 (m, H-C(4')); 3.82 (m, CH₂ (5')); 2.87 (m, H_{α}-C(2')); 2.20 (dd, J = -15.4, 2.0, H_{β}-C(2')); 1.69 (s, Me). Anal. calcd. for C₁₇H₁₈N₂O₆ (346.34): C 58.96, H 5.24, N 8.09; found: C 59.11, H 5.35 N 7.99.

### Beispiel 39

### 1-(2-Deoxy-β-D-threo-pentofuranosyl)thymine 5'-Monophosphate, Triethylammonium (8a).

### Methode A:

Die Lösung von Verbindung 7a) (80 mg, 0,23 mmol) in PO(0Me)₃(0,5 ml) wird mit POCl₃ (46 µl, 0,48 mmol) bei 4°C für 8 Stunden inkubiert. Die Reaktionsmischung wird in kaltes 1 molares wäßriges Triethylammoniumbicarbonat (15 ml) gegeben, zwei Stunden bei Raumtemperatur inkubiert, eingedampft und nochmals zweimal mit Wasser (50 ml) aufgenommen und eingedampft. Der Rest wird mit 25 %iger wäßriger Ammoniaklösung (50 ml) für drei Stunden inkubiert, eingedampft und an DEAE Sephadex (HCO₃⁻ Form, 2 x 30 cm) chromatographiert. Die Elution wird mit Wasser (600 ml) und dann mit einem linearen Gradienten von Triethylammoniumbicarbonat (0 - 0,3 mol/l, 1200 ml) durchgeführt. Die Phosphat-enthaltenden Fraktionen eluieren bei 0,2 - 0,25 mol/l. Sie werden eingedampft, nochmals in Wasser aufgenommen und eingedampft und nochmals an DEAE Sephadex unter den gleichen Bedingungen chromatographiert. Triethylammoniumbicarbonat wird durch wiederholtes Eindampfen mit Wasser und Ethanol entfernt. Es wird ein farbloses amorphes Produkt erhalten.

(1443 A₂₆₇ units, 71%). TLC (EtOAc/acetone/EtOH/water, 15:3:4:3): R_{f} 0.06, (2-propanol/25% aq. ammonia/water, 7:1:1): R_{f} 0.10. HPLC (0.1 M Triethylammonium acetate/5% MeCN; 0.6 ml/min): t_{R} 8 min.
Electrophoresis: E_{UP} 0.87. UV (H₂O): ₘₐₓ 267 nm. ³¹P-NMR (D₂O/0.1 M Tris.HCl buffer): 1.36.

### Methode B:

### 9-[2-Deoxy-5-O-(4,4'-dimethoxytriphenylmethyl)-β-D-threo-pentofuranosyl]adenine (5b).

Die Verbindung wird aus Verbindung 7a) (80 mg, 0,23 mmol), wie bei Methode A erhalten. Lediglich die Entfernung der Benzoylgruppe wird durchgeführt mit 0,1 mol/l NaOH in 50 % wäßrigem Methanol (15 ml) bei 50°C in 2 Stunden. Die Lösung wird mit Dowex 50 (H⁺ Form) neutralisiert, der Ionentauscher abfiltriert und die neutrale Lösung eingedampft. Die weitere Reinigung erfolgt wie in Methode A beschrieben. Es werden 1214 A₂₇₆ units (60 %) erhalten.

### Beispiel 40

Verbindung 3 (300 mg, 1,19 mmol) in Pyridin (15 ml) wird mit 4,4'-Dimethoxytritylchlorid (508 mg, 1,5 mmol) und Ethyldiisopropylamin (255 µl, 1,5 mmol) 12 Stunden bei Raumtemperatur inkubiert. Die Lösung wird bei 30°C eingedampft. Der Rest wird nochmals mit in Toluol aufgenommen, eingedampft und an Silikagel (4 x 11 cm) chromatographiert.
CH₂Cl₂/acetone/Et₃N (40:40:3). White foam (476 mg, 72 %). TLC (CH₂Cl₂/acetone/Et₃N, 40:40:3): R_{f} 0.64. ¹H-NMR ((D₆)DMSO): 8.26 (s, H-C(8)); 8.16 (s, H-C(2)); 7.41 - 7.18 (m, 9 arom. H, NH₂); 6.84 - 6.77 (m, 4 arom. H); 6.35 (d, J = 8.2, H-C(1')); 5.94 (d, J = 5.2, OH-C(3')); 4.32 (m, H-C(3')); 4.19 (m, H-C(4')); 3.71 (s, 2 OMe); 3.23-3.02 (m, CH₂-(5'); 2.78 (m, H_{α}-C(2')); 2.29 (d, J = - 14.8, H_{β}- C(2')).

### Beispiel 41

### 9-[3-O-Benzoyl-2-deoxy-5-O-(4,4'-dimethoxytriphenylmethyl)-β-D-threo-pentofuranosyl]adenine (6b).

Die Lösung von Verbindung 5b) (400 mg, 0,72 mmol) in MeCN (4 ml) wird mit Benzoylcyanid (105 mg, 0,8 mmol) und Triethylamin (130 µl, 0,39 mmol) bei Raumtemperatur 3 Stunden inkubiert. Es werden zusätzlich 25 mg (0,19 mmol) Benzoylcyanid, 25 µl Triethylamin zugegeben und weitere 15 Stunden gerührt. Die Lösung wird eingedampft und der Rest an Silikagel (2 x 15 cm) chromatographiert, mit CH₂Cl₂/acetone (1:2) to give a white foam (305 mg, 64%). TLC (CH₂Cl₂/acetone, 1:2): R_{f} 0.60. ¹H-NMR ((D₆)DMSO: 8.09 (s, H-C(8)); 8.07 (s, H-C(2)); 7.68-7.13 (m, 14 arom. H, NH₂); 6.75 - 6.69 (m, 4 arom. H); 6.41 (d, J = 5.2, H-C(1')); 5.82 (m, H-C(3')); 4.57 (m, H-C(4')); 3.69 (s, OMe); 3.67 (s, OMe); ca 3.3 - 3.2 (m, CH₂(5'); 3.05 (m, H_{α} -C(2')); 2.91 (d, J = - 14.3, H_{β}-C(2')).

### Beispiel 42

### 9-(3-O-Benzoyl-2-deoxy-β-D-threo-pentofuranosyl)adenine (7b).

Verbindung 6b) (260 mg, 0,40 mmol) wird in 80 %iger Essigsäure (10 ml) aufgenommen und für 80 Minuten bei Raumtemperatur gerührt. Die Lösung wird mit 50 ml Wasser verdünnt und eingedampft. Der Rest wird je zweimal mit 20 ml Wasser und einmal mit 20 ml 2-Propanol aufgenommen und eingedampft. Das Produkt wird an Silikagel (2 x 12 cm) gereinigt und chromatographiert in CH₂Cl₂/MeOH 93:7. Colourless solid (120 mg, 85%). M.p. 166-168°C (2-propanol). TLC (CH₂Cl₂/MeOH, 93:7): R_{f} 0.30. ¹H-NMR ((D₆)DMSO): 8.28. (s, H-C(8)); 8.07 (s, H-C(2)); 7.85 - 7.47 (m, 5 arom. H); 7.27 (s, NH₂); 6.38 (d, J = 5.2, H-C(1')); 5.68 (m, H-C(3')); 5.01 (br, OH-C(5')); 4.33 (m, H-C(4')); 3.76 (m, H-C(5')); 3.02 (m, H_{α}-C(2')); 2.83 (d, J = -14.7, H_{β}-C(2')). Anal. calcd. for C₁₇H₁₇N₅O₄ (355.35): C 57.46, H 4.82, N 19.71; found: C 57.15, H 5.02, N 19.49.

### Beispiel 43

### 9-(2-Deoxy-β-D-threo-pentofuranosyl)adenine 5'-Phosphate, Triethylammonium (8b).

Verbindung 8b) wird aus Verbindung 7b) (53 mg, 0,15 mmol) wie in Methode A zur Herstellung von Verbindung 8a) hergestellt. Die Zeit für die POCl₃-Behandlung beträgt 4 Stunden. Es wird ein amorphes Produkt (1222 A₂₅₈ units, 53 %) erhalten. Das Produkt wird in ca 200 µl Methanol gelöst und mit 20 ml Ether präzipitiert. Das Präzipitat wird durch Zentrifugation abgetrennt und über Phosphopentoxid getrocknet. Dünnschichtchromatographie erfolgt mit 2-propanol/25% ammonia/water, 7:1:2): R_{f} 0.24, (EtOAc/acetone/EtOH/water, 15:3:4:3): R_{f} 0.06. HPLC (0.1 M Triethylammonium acetate/5% MeCN; 0.6 ml/min) t_{R} 13 min. Electrophoresis: E_{UP} 0.68. UV (MeOH): ₘₐₓ 259 nm. ³¹P-NMR (D₂O/MeOD, 1:1): 2.94. ¹H-NMR (D₂O/MeOD, 1:1): 9.89 (s, H-C(8)); 9.71 (s, H-C(2)); 7.84 (d, J = 8.0, H-C(1')); 6.08 (m, H-C(3')); 5.77 - 5.5.61 (m, CH₂(5')); 5.01 (m, H-C(4')); 4.68 (q, CH₂); 4.48 (m, H_{α}-C(2')); 3.92 (d, H_{β}-C(2')); 2.74 (m, CH₃).

### Beispiel 44

### Verbindung 5a) wird analog

Rosemeyer, H.; Seela, F. *Helv. Chim. Acta* 1991*, 74,* 748.

Rosemeyer, H.; Krecmerova M.; Seela F. *Helv. Chim*. *Acta*, 1991, *74*, 2054.

Fox, J.J.; Miller, N. C. *J. Org. Chem.* 1963, *28,* 936.

### hergestellt.

Daten von Verbindung 4a:
824 A₂₆₀ units (53.8%) of colourless solid. TLC (2-propanol/25% aqueous ammonia/water, 7:1:1): R_{f} 0.54. HPLC (5% MeCN in 0.1M triethylammonium acetate, pH 7, 0.6 ml/min): t_{R} 15 min.
Electrophoresis: E_{UP} 0.35. UV (MeOH): ₘₐₓ 260 nm. ³¹P-NMR (D₂O/0.1 M Tris-HCl buffer, 1:1): - 5.11 (d, J = 19.4). ¹H-NMR ((D₆)DMSO): 9.97 (br, NH); 8.33 (s, H-C(8)): 8.15 (s, H-C(2)); 7.34 (s, NH₂); 6.40 (d, J = 7.8, H-C(1')); 4.63 (br, H-C(3')); 4.42 - 4.10 (m, CH₂ (5')); 3.94 (br, H-C(4')); 3.05 (q, CH₂); 2.85 (m, H_{α}-C(2')); 2.28 (d, J = -14.6, H_{β}-C(2')); 1.18 (t, Me).

## Patentansprüche

1. Oligodesoxyribonukleotide, in denen jeweils am 5'- und 3'-Ende mindestens zwei 2'-Desoxy-β-D-erythropentofuranosyl-Gruppen durch 2'-Desoxy-β-D-threo-pentofuranosyl-Gruppen ersetzt sind, und die aus 6 bis 100 Nukleotidbausteinen bestehen.

2. Oligodesoxyribonukleotide, in denen mindestens 20 % der 2'-Desoxy-β-D-erythro-pentofuranosyl-Gruppen in aufeinanderfolgenden Nukleotidbausteinen durch 2'-Desoxy-β-D-threo-pentofuranosyl-Gruppen ersetzt sind und die aus 6 - 100 Nukleotidbausteinen bestehen.

3. Oligodesoxyribonukleotide nach Anspruch 1 oder 2, dadurch gekennzeichnet, daß sie aus 15 bis 30 Nukleotidbausteinen bestehen.

4. Oligodesoxyribonukleotide nach den Ansprüchen 1 bis 3, daduch gekennzeichnet, daß mindestens 30 % der 2'-Desoxy--β-D-erythro-pentofuranosyl-Gruppen durch 2'-Desoxy-β-D-threo-pentofuranosyl-Gruppen ersetzt sind.

5. Oligodesoxyribonukleotide nach den Ansprüchen 1 bis 4, dadurch gekennzeichnet, daß alle 2'-Desoxy-β-D-erythropentofuranosyl-Gruppen durch 2'-Desoxy-β-D-threo-pentofuranosyl-Gruppen ersetzt sind.

6. Oligodesoxyribonukleotide nach den Ansprüchen 1 bis 5 dadurch gekennzeichnet, daß einer oder mehrere Nukleotidbausteine an Erkennungssequenzen für Endonukleasen im Oligonukleotid durch 2'-Desoxyxylonukleotide ersetzt sind.

7. Oligodesoxyribonukleotide nach den Ansprüchen 1 - 6, dadurch gekennzeichnet, daß sie mindestens eine Base aus der Gruppe 1-Desazaadenin, 3-Desazaadenin, 7-Desazaadenin, 1-Desazaguanin, 3-Desazaguanin, 7-Desazaguanin, 1-Desazahypoxanthin, 3-Desazahypoxanthin, 7-Desazahypoxanthin, an C-7 substituierte 7-Desazapurine, an C-8 substituierte Purine, C-5 substituierte Pyrimidine enthalten.

8. Oligodesoxyribonukleotide nach den Ansprüchen 1 - 7, dadurch gekennzeichnet, daß sie an dem 5'- und/oder 3'-Ende am 5'-Ende Wasserstoff, eine Reportergruppe oder eine Intercalatorgruppe enthalten.

9. Oligonukleotide nach Anspruch 8, dadurch gekennzeichnet, daß sie am 5'-Ende eine Mono-, Di- oder Triphosphatgruppe tragen.

10. Verfahren zur Herstellung von Oligodesoxyribonukleotiden, in denen jeweils am 5'- und 3'-Ende mindestens zwei 2'-Desoxy-β-D-erythro-pentofuranosyl-Gruppen durch 2'-Desoxy-β-D-threo-pentofuranosylgruppen ersetzt sind, und aus 6 bis 100 Nukleotidbausteinen bestehen, nach dem Verfahren der Oligonukleotidsynthese, wobei ein Startnukleosid an einen festen Träger gebunden wird und anschließend durch schrittweise Kupplung mit entsprechend aktivierten monomeren Nukleotidbausteinen das gewünschte Oligonukleotid aufgebaut wird, ggf. während und nach der Synthese dreiwertiger Phosphor zu fünfwertigem Phosphor oxidiert wird, das Oligonukleotid vom Träger mit einer ersten Base, heterocyklische Schutzgruppen mit einer zweiten Base, die 5'-Schutzgruppe mit einer Säure abgespalten und das Oligonukleotid ggf. gereinigt wird.

11. Verfahren zur Herstellung von Oligodesoxyribonukleotiden in denen mindestens 20 % der β-D-2'-Desoxy-erythropentofuranosyl-Gruppen in aufeinanderfolgenden Nukleotidbausteinen durch β-D-2'-Desoxy-threo-pentofuranosyl-Gruppen ersetzt sind und die aus 6 - 100 Nukleotidbausteinen bestehen nach dem Verfahren der Oligonukleotidsynthese, wobei ein Startnukleosid an einen festen Träger gebunden wird und anschließend durch schrittweise Kupplung mit entsprechend aktivierten monomeren Nukleotidbausteinen das gewünschte Oligonukleotid aufgebaut wird, ggf. während und nach der Synthese dreiwertiger Phosphor zu fünfwertigem Phosphor oxidiert wird, das Oligonukleotid vom Träger mit einer ersten Base, heterocyklische Schutzgruppen mit einer zweiten Base, mit einer Säure die 5'-Schutzgruppe abgespalten und das Oligonukleotid ggf. aufgereinigt wird.

12. 2'-Desoxy-xylonukleosid-3'-0-phosphoramidite

13. 2'-Desoxy-β-D-xylonukleosid-3'-0-H-phosphonate und deren Salze

14. 2'-Desoxy-β-D-xylonukleosid-P-methylphosphoamidite.

15. Nukleotide der allgemeinen Formel 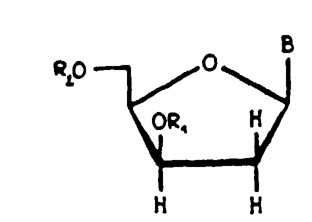 wobei B entweder A, T, C, G oder eine Base aus der Gruppe 1-Desazaadenin, 3-Desazaadenin, 7-Desazaadenin, 1-Desazaguanin, 3-Desazaguanin, 7-Desazaguanin, 1-Desazahypoxanthin, 3-Desazahypoxanthin, 7-Desazahypoxanthin, an C-7 substituierte 7-Desazapurine, an C-8 substituierte Purine, C-5 substituierte Pyrimidine enthält, R₁ ein Wasserstoffatom oder eine Reportergruppe oder eine Intercalatorgruppe und R₂ ein Mono-, Di oder Triphosphat darstellt.

16. Verbindungen nach den Ansprüchen 12 bis 15, dadurch gekennzeichnet, daß sie an 5'-O und/oder an der heterocyclischen Base Schutzgruppen tragen.

17. Verwendung von Oligodesoxyribonukleotiden, in denen jeweils am 5'- und 3'-Ende mindestens zwei 2'-Desoxy-β-D-erythro-pentafuranosyl-Gruppen durch 2'-Desoxy-β-D-threo-pentafuranosyl-Gruppen ersetzt sind und die aus 6 bis 100 Nukleotidbausteinen bestehen, zur Herstellung eines Arzneimittels mit antiviraler Wirksamkeit.

18. Verwendung von Oligodesoxyribonukleotiden, in denen mindestens 20 % der 2'-Desoxy-β-D-erythro-pentofuranosyl-Gruppen in aufeinanderfolgenden Nukleotidbausteinen durch 2'-Desoxy-β-D-threo-pentofuranosyl-Gruppen ersetzt sind und die aus 6 - 100 Nukleotidbausteinen bestehen, zur Herstellung eines Arzneimittels mit antiviraler Wirksamkeit.

19. Desoxyxylonucleosid-5'-Mono-,Di- und Triphosphate.

20. Verfahren zur Herstellung von Verbindungen nach Anspruch 19, welches dadurch gekennzeichnet ist, daß das entsprechende Desoxyxylonucleosid an der 3'OH-Gruppe benzoyliert und anschließend mit POCl₃ in Trialkylphosphat zum 5'-Phosphat phosphoryliert und die 3'-Schutzgruppe mit einer Base entfernt wird.

## Claims

1. Oligodeoxyribonucleotides in which at least two 2'-deoxy-β-D-erythro-pentofuranosyl groups are replaced by 2'-deoxy-β-D-threo-pentofuranosyl groups at both the 5' end and 3' end and which are composed of 6 to 100 nucleotide building blocks.

2. Oligodeoxyribonucleotides in which at least 20 % of the 2'-deoxy-β-D-erythro-pentofuranosyl groups in consecutive nucleotide building blocks are replaced by 2'-deoxy-β-D-threo-pentofuranosyl groups and which are composed of 6 to 100 nucleotide building blocks.

3. Oligodeoxyribonucleotides as claimed in claim 1 or 2, wherein they are composed of 15 to 30 nucleotide building blocks.

4. Oligodeoxyribonucleotides as claimed in claims 1 to 3, wherein at least 30 % of the 2'-deoxy-β-D-erythro-pentofuranosyl groups are replaced by 2'-deoxy-β-D-threo-pentofuranosyl groups.

5. Oligodeoxyribonucleotides as claimed in claims 1 to 4, wherein all 2'-deoxy-β-D-erythro-pentofuranosyl groups are replaced by 2'-deoxy-β-D-threo-pentofuranosyl groups.

6. Oligodeoxyribonucleotides as claimed in claims 1 to 5, wherein one or several nucleotide building blocks are replaced by 2'-deoxyxylonucleotides at recognition sequences for endonucleases in the oligonucleotide.

7. Oligodeoxyribonucleotides as claimed in claims 1-6, wherein they contain at least one base from the group comprising 1-deazaadenine, 3-deazaadenine, 7-deazaadenine, 1-deazaguanine, 3-deazaguanine, 7-deazaguanine, 1-deazahypoxanthine, 3-deazahypoxanthine, 7-deazahypoxanthine, 7-deazapurines substituted at C-7, purines substituted at C-8, pyrimidines substituted at C-5.

8. Oligodeoxyribonucleotides as claimed in claims 1 - 7, wherein they contain hydrogen, a reporter group or an intercalator group at the 5' end and/or 3' end.

9. Oligonucleotides as claimed in claim 8, wherein they carry a mono, di or triphosphate group at the 5' end.

10. Process for the production of oligodeoxyribonucleotides in which at least two 2'-deoxy-β-D-erythro-pentofuranosyl groups are replaced by 2'-deoxy-β-D-threo-pentofuranosyl groups at both the 5' end and 3' end and are composed of 6 to 100 nucleotide building blocks, by a process of oligonucleotide synthesis in which a start nucleoside is bound to a solid support and subsequently the desired oligonucleotide is synthesized by stepwise coupling with appropriately activated monomeric nucleotide building blocks, if desired trivalent phosphorus is oxidized to pentavalent phosphorus during and after the synthesis, the oligonucleotide is cleaved from the support using a first base, heterocyclic protecting groups are cleaved with a second base, the 5' protecting group is cleaved with an acid and the oligonucleotide is purified if desired.

11. Process for the production oligodeoxyribonucleotides in which at least 20 % of the β-D-2'-deoxy-erythro-pentofuranosyl groups in consecutive nucleotide building blocks are replaced by β-D-2'-deoxy-β-D-threo-pentofuranosyl groups and which are composed of 6 to 100 nucleotide building blocks by a process of oligonucleotide synthesis, in which a start nucleoside is bound to a solid support and subsequently the desired oligonucleotide is synthesized by stepwise coupling with appropriately activated monomeric nucleotide building blocks, if desired trivalent phosphorus is oxidized to pentavalent phosphorus during and after the synthesis, the oligonucleotide is cleaved from the support using a first base, heterocyclic protecting groups are cleaved with a second base, the 5' protecting group is cleaved with an acid and the oligonucleotide is purified if desired.

12. 2'-Deoxy-xylonucleoside-3'-O-phosphoramidites.

13. 2'-Deoxy-β-D-xylonucleoside-3'-O-H-phosphonates and their salts.

14. 2'-Deoxy-β-D-xylonucleoside-P-methylphosphoramidites.

15. Nucleotides having the general formula 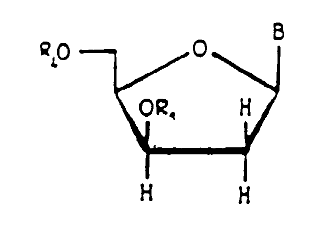 in which B represents either A, T, C, G or a base from the group comprising 1-deazaadenine, 3-deazaadenine, 7-deazaadenine, 1-deazaguanine, 3-deazaguanine, 7-deazaguanine, 1-deazahypoxanthine, 3-deazahypoxanthine, 7-deazahypoxanthine, 7-deazapurines substituted at C-7, purines substituted at C-8, pyrimidines substituted at C-5, R₁ represents a hydrogen atom or a reporter group or an intercalator group and R₂ represents a mono, di or triphosphate.

16. Compounds as claimed in claims 12 to 15, wherein they carry protecting groups at the 5'-O and/or on the heterocyclic base.

17. Use of oligodeoxyribonucleotides in which at least two 2'-deoxy-β-D-erythro-pentofuranosyl groups are replaced by 2'-deoxy-β-D-threo-pentofuranosyl groups at both the 5' end and 3' end and which are composed of 6 to 100 nucleotide building blocks for the production of a pharmaceutical agent with antiviral activity.

18. Use of oligodeoxyribonucleotides in which at least 20 % of the 2'-deoxy-β-D-erythro-pentofuranosyl groups in consecutive nucleotide building blocks are replaced by 2'-deoxy-β-D-threo-pentofuranosyl groups and which are composed of 6 to 100 nucleotide building blocks for the production of a pharmaceutical agent with antiviral activity.

19. Deoxyxylonucleoside-5'-mono, di and triphosphates.

20. Process for the production of compounds as claimed in claim 19, wherein the 3'OH group of the corresponding deoxyxylonucleoside is benzoylated and subsequently phosphorylated with POCl₃ in trialkylphosphate to form 5'-phosphate and the 3' protecting group is removed with a base.

## Revendications

1. Oligodésoxyribonucléotides, dans lesquels, à chacune des extrémités 5' et 3', au moins deux groupes 2'-désoxy-β-D-érythropentofuranosyle sont remplacés par des groupes 2'-désoxy-β-D-thréopentofuranosyle et qui sont constitués par 6 à 100 nucléotides.

2. Oligodésoxyribonucléotides, dans lesquels au moins 20% des groupes 2'-désoxy-β-D-érythro-pentofuranosyle présents dans des nucléotides successifs sont remplacés par des groupes 2'-désoxy-β-D-thréo-pentofuranosyle et qui sont constitués par 6 à 100 nucléotides.

3. Oligodésoxyribonucléotides selon la revendication 1 ou 2, caractérisés en ce qu'ils sont consitués par 15 à 20 nucléotides.

4. Oligodésoxyribonucléotides selon les revendications 1 à 3, caractérisés en ce qu'au moins 30 % des groupes 2'-désoxy-β-D-érythropentofuranosyle sont remplacés par des groupes 2'-désoxy-β-D-thréo-pentofuranosyle.

5. Oligodésoxyribonucléotides selon les revendications 1 à 4, caractérisés en ce que tous les groupes 2'-désoxy-β-D-érythropentofuranosyle sont remplacés par des groupes 2'-désoxy-β-D-thréo-pentofuranosyle.

6. Oligodésoxyribonucléotides selon les revendications 1 à 5, caractérisés en ce qu'un ou plusieurs nucléotides présents dans des séquences reconnues par des endonucléases au sein d'un oligonucléotide sont remplacés par des 2'-désoxyxylonucléotides.

7. Oligodésoxyribonucléotides selon les revendications 1 à 6, caractérisés en ce qu'ils contiennent au moins une base appartenant au groupe formé par la 1-désaza-adénine, la 3-désaza-adénine, la 7-désaza-adénine, la 1-désazaguanine, la 3-désazaguanine, la 7-désazaguanine, la 1-désazahypoxanthine, la 3-désazahypoxanthine, la 7-désazahypoxanthine, la 7-désazapurine substituée en C-7, la purine substituée en C-8, la pyrimidine substituée en C-5.

8. Oligodésoxyribonucléotides selon les revendications 1 à 7, caractérisés en ce qu'ils contiennent, à l'extrémité 5' et/ou 3', un atome d'hydrogène, un groupe rapporteur ou un groupe intercalaire.

9. Oligodésoxyribonucléotides selon la revendication 8, caractérisés en ce qu'à l'extrémité 5', ils portent un groupe mono-, di- ou triphosphate.

10. Procédé de préparation d'oligonucléotides dans lesquels, à chacune des extrémités 5' et 3', au moins deux groupes 2'-désoxy-β-D-érythro-pentofuranosyle sont remplacés par des groupes 2'-désoxy-β-D-thréo-pentofuranosyle et qui sont constitués par 6 à 100 nucléotides, selon le procédé de synthèse des oligonucléotides, dans lequel un nucléoside de départ est fixé sur un support solide et ensuite, au moyen d'un couplage par étapes avec des nucléotides monomères activés de manière appropriée, l'oligonucléotide souhaité est formé, éventuellement pendant et après la synthèse, le phosphore trivalent est oxydé en phosphore pentavalent, l'oligonucléotide est coupé du support à l'aide d'une première base, les groupes protecteurs hétérocycliques sont coupés à l'aide d'une seconde base, les groupes protecteurs en 5' sont coupés à l'aide d'un acide et l'oligonucléotide est éventuellement purifié.

11. Procédé de préparation d'oligonucléotides dans lesquels au moins 20 % des groupes 2'-désoxy-β-D-érythro-pentofuranosyle présents dans des nucléotides successifs sont remplacés par des groupes 2'-désoxy-β-D-thréo-pentofuranosyle et qui sont constitués par 6 à 100 nucléotides, selon le procédé de synthèse des oligonucléotides, dans lequel un nucléoside de départ est fixé sur un support solide et ensuite, au moyen d'un couplage par étapes avec des nucléotides monomères activés de manière appropriée, l'oligonucléotide souhaité est formé, éventuellement pendant et après la synthèse, le phosphore trivalent est oxydé en phosphore pentavalent, l'oligonucléotide est coupé du support à l'aide d'une première base, les groupes protecteurs hétérocycliques sont coupés à l'aide d'une seconde base, les groupes protecteurs en 5' sont coupés à l'aide d'un acide et l'oligonucléotide est éventuellement purifié.

12. 2'-désoxy-xylonucléoside-3'-O-phosphoramidite

13. 2'-désoxy-β-D-xylonucléoside-3'-O-H-phosphonate et ses sels.

14. 2'-désoxy-β-D-xylonucléoside-P-méthylphosphoramidite.

15. Nucléotides de formule générale 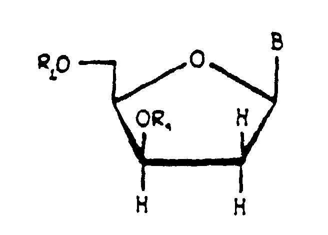 dans laquelle B contient soit A, T, C, G soit une base appartenant au groupe formé par la 1-désaza-adénine, la 3-désaza-adénine, la 7-désaza-adénine, la 1-désazaguanine, la 3-désazaguanine, la 7-désazaguanine, la 1-désazahypoxanthine, la 3-désazahypoxanthine, la 7-désazahypoxanthine, la 7-désazapurine substituée en C-7, la purine substituée en C-8, la pyrimidine substituée en C-5, R₁ représente un atome d'hydrogène, un groupe rapporteur ou un groupe intercalaire et R₂, un mono-, di- ou triphosphate.

16. Composés selon les revendications 12 à 15, caractérisés en ce qu'ils portent des groupes protecteurs en 5'-O et/ou sur la base hétérocyclique.

17. Utilisation d'oligodésoxyribonucléotides dans lesquels, à chacune des extrémités 5' et 3', au moins deux groupes 2'-désoxy-β-D-érythro-pentofuranosyle sont remplacés par des groupes 2'-désoxy-β-D-thréo-pentofuranosyle et qui sont constitués par 6 à 100 nucléotides, pour la préparation de médicaments ayant une activité antivirale.

18. Utilisation d'oligodésoxyribonucléotides dans lesquels au moins 20 % des groupes 2'-désoxy-β-D-érythro-pentofuranosyle présents dans des nucléotides successifs sont remplacés par des groupes 2'-désoxy-β-D-thréo-pentofuranosyle et qui sont constitués par 6 à 100 nucléotides, pour la préparation de médicaments ayant une activité antivirale.

19. Désoxyxylonucléoside-5'-mono-, di- ou triphosphates.

20. Procédé de préparation de composés selon la revendication 19, caractérisé en ce que le désoxyxylonucléoside correspondant est benzoylé sur le groupe 3'-OH et ensuite, il est phosphorylé en 5'-phosphate avec POCl₃ dans du phosphate de trialkyle et le groupe protecteur en 3' est éliminé à l'aide d'une base.
